(19) Europäisches Patentamt
European Patent Office
Office européen des brevets

(11) Veröffentlichungsnummer: **0 499 096 A1**

(12) **EUROPÄISCHE PATENTANMELDUNG**

(21) Anmeldenummer: **92101511.1**

(22) Anmeldetag: **30.01.92**

(51) Int. Cl.[5]: **C07D 251/16**, A01N 47/44, C07D 403/12, C07D 251/46, C07D 251/42, C07D 409/12

(30) Priorität: **12.02.91 DE 4104154**

(43) Veröffentlichungstag der Anmeldung: **19.08.92 Patentblatt 92/34**

(84) Benannte Vertragsstaaten: **BE CH DE DK ES FR GB IT LI NL**

(71) Anmelder: **BAYER AG**

**W-5090 Leverkusen 1 Bayerwerk(DE)**

(72) Erfinder: **Gesing, Ernst, Dr.**
**Trillser Graben 4**
**W-4006 Erkrath-Hochdahl(DE)**
Erfinder: **Fest, Christa, Dr.**
**Im Johannistal 20**
**W-5600 Wuppertal(DE)**
Erfinder: **Kirsten, Rolf, Dr.**
**Carl-Langhans-Strasse 27**
**W-4019 Monheim(DE)**
Erfinder: **Kluth, Joachim, Dr.**
**Tannenweg 9**
**W-4018 Langenfeld(DE)**
Erfinder: **Müller, Klaus-Helmut, Dr.**
**Bockhackstrasse 55**
**W-4000 Düsseldorf 13(DE)**
Erfinder: **Riebel, Hans-Jochem, Dr.**
**In der Beek 92**
**W-5600 Wuppertal(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen(DE)**
Erfinder: **Schmidt, Robert R., Dr.**
**Im Waldwinkel 110**
**W-5060 Bergisch Gladbach 2(DE)**

(54) **Substituierte Arylsulfonylaminoguanidinotriazine.**

(57) Die Erfindung betrifft neue substituierte Arylsulfonylaminoguanidinotriazine der allgemeinen Formel (I)

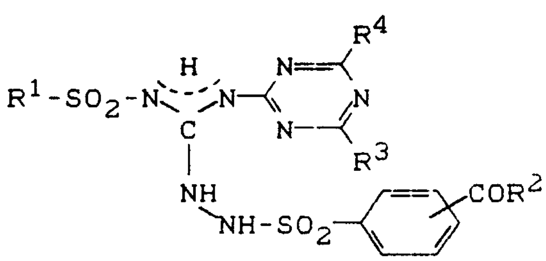

(I)

in welcher
$R^1$ für jeweils gegebenenfalls substituiertes Aryl, Aralkyl oder Heteroaryl steht,
$R^2$ für Hydroxy, Amino oder für jeweils gegebenenfalls substituiertes Alkoxy oder Alkylamino steht,
$R^3$ für Halogen, Alkyl, Halogenalkyl, Alkoxyalkyl, Alkoxy, Halogenalkoxy, Alkylthio, Halogenalkylthio, Alkylamino oder Dialkylamino steht und
$R^4$ für Halogen, Alkyl, Cycloalkyl, Alkoxy, Alkylthio, Alkylamino oder Dialkylamino steht,
wobei N'-(4,6-Dimethoxy-s-triazin-2-yl)-N''-(2-methoxycarbonyl-phenylsulfonylamino)-N'''-(2-brom phenylsulfonyl)-guanidin, N'-(4,6-Dimethoxy-s-triazin-2-yl)-N''-(2-methoxycarbonyl-phenylsulfonylamino)-N'''-(2-phenyl-phe-

EP 0 499 096 A1

nylsulfonyl)-guanidin, N'-(4-Methoxy-6-methyls-triazin-2-yl)-N''-(2-methoxycarbonyl-phenylsulfonylamino)-N'''-(2-phenyl-phenylsulfonyl)-guanidin, N'-(4,6-Dimethoxy-s-triazin-2-yl)-N''-(2-methoxycarbonyl-phenylsulfonylamino)-N'''-(2-methylthio-phenylsulfonyl)-guanidin und N-(4,6-Dimethoxy-s-triazin-2-yl)-N''-(2-methoxycarbonyl-phenylsulfonylamino)-N'''-(2-isopropoxycarbonyl-phenylsulfonyl)-guanidin ausgenommen sind,
Verfahren zu ihrer Herstellung und ihre Verwendung als Herbizide.

Die Erfindung betrifft neue substituierte Arylsulfonylaminoguanidinotriazine, Verfahren zu ihrer Herstellung und ihre Verwendung als Herbizide.

Es ist bereits bekannt, daß bestimmte Sulfonylaminoguanidinoazine, wie z.B. N'-(4,6-Dimethyl-pyrimidin-2-yl)-N''-(4-methyl-phenylsulfonylamino)-N'''-(2-chlor-phenylsulfonyl)-guanidin und N'-(4,6-Dimethyl-pyrimidin-2-yl)-N''-(2-methoxycarbonyl-phenylsulfonylamino)-N'''-(2-methoxycarbonyl-phenylsulfonyl)-guanidin, herbizide Eigenschaften aufweisen (vgl. EP-A 121082 und EP-A 302378). Die Herbizidwirkung dieser bekannten Verbindungen ist jedoch nicht in allen Belangen zufriedenstellend.

Weitere substituierte Arylsulfonylaminoguanidinoazine sind Gegenstand einer vorgängigen, jedoch nicht vorveröffentlichten Patentanmeldung (vgl. DE-P 3927770 vom 23.08.1989).

Es wurden nun neue substituierte Arylsulfonylaminoguanidinotriazine der allgemeinen Formel (I)

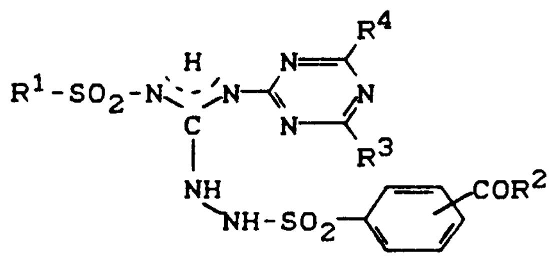

(I)

in welcher

$R^1$ für jeweils gegebenenfalls substituiertes Aryl, Aralkyl oder Heteroaryl steht,

$R^2$ für Hydroxy, Amino oder für jeweils gegebenenfalls substituiertes Alkoxy oder Alkylamino steht,

$R^3$ für Halogen, Alkyl, Halogenalkyl, Alkoxyalkyl, Alkoxy, Halogenalkoxy, Alkylthio, Halogenalkylthio, Alkylamino oder Dialkylamino steht und

$R^4$ für Halogen, Alkyl, Cycloalkyl, Alkoxy, Alkylthio, Alkylamino oder Dialkylamino steht,

gefunden, wobei N'-(4,6-Dimethoxy-s-triazin-2-yl)-N''-(2-methoxycarbonyl-phenylsulfonylamino)-N'''-(2-bromphenylsulfonyl)-guanidin, N'-(4,6-Dimethoxy-s-triazin-2-yl)-N''-(2-methoxycarbonyl-phenylsulfonylamino)-N'''-(2-phenyl-phenylsulfonyl)-guanidin, N'-(4-Methoxy-6-methyl-s-triazin-2-yl)-N''-(2-methoxycarbonyl-phenylsulfonylamino)-N'''-(2-phenyl-phenylsulfonyl)-guanidin, N'-(4,6-Dimethoxy-s-triazin-2-yl)-N''-(2-methoxycarbonyl-phenyl sulfonylamino)-N'''-(2-methylthio-phenylsulfonyl)-guanidin und N-(4,6-Dimethoxy-s-triazin-2-yl)-N''-(2-methoxycarbonyl-phenylsulfonylamino)-N'''-(2-isopropoxycarbonyl-phenylsulfonyl)-guanidin ausgenommen sind (vgl. DE-P 3927770 bzw. EP-Anm.Nr. 90115373.4/EP-A-0414067).

Die allgemeine Formel (I) steht für die einzelnen möglichen Tautomeren der Formeln (IA), (IB) und (IC)

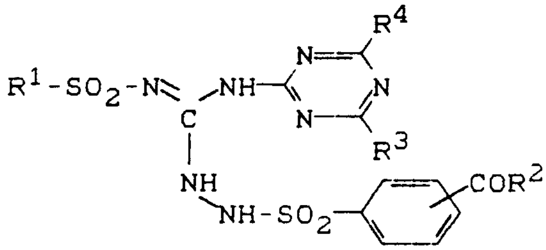

(IA)

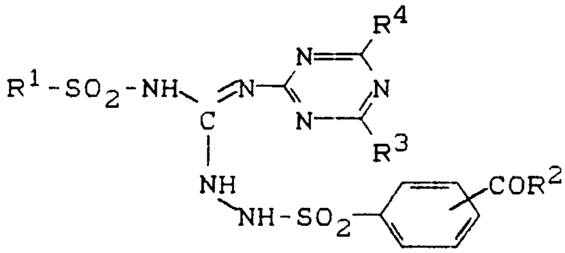

(IB)

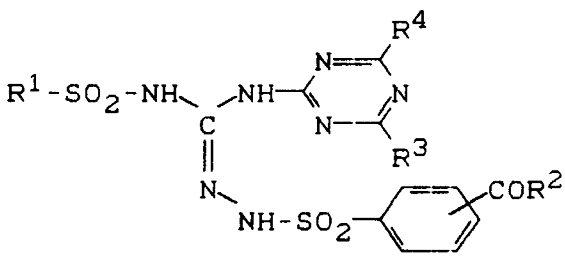

(IC)

sowie für Gemische dieser Tautomeren.

Man erhält die neuen substituierten Arylsulfonylaminoguanidinoazine der allgemeinen Formel (I), wenn man

(a) Sulfonylverbindungen der allgemeinen Formel (II)

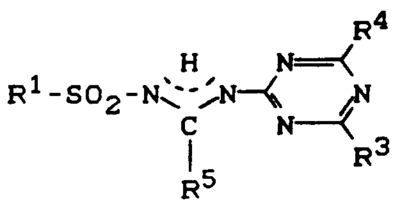

(II)

in welcher

| | |
|---|---|
| $R^1$, $R^3$ und $R^4$ | die oben angegebenen Bedeutungen haben und |
| $R^5$ | für eine der nachstehend angegebenen Abgangsgruppen |

$$R^6-SO_2-\underset{|}{N}-OR^7 \text{ oder } -Q-R^8 \text{ steht,}$$

worin

| | |
|---|---|
| $R^6$ | die oben für $R^1$ angegebene Bedeutung hat, aber nicht in jedem Einzelfall mit $R^1$ identisch sein muß, |
| $R^7$ | für Alkyl, Alkenyl oder Aralkyl steht, |
| $R^8$ | für Alkyl, Aralkyl oder Aryl steht und |
| Q | für Sauerstoff oder Schwefel steht, mit Sulfonsäurehydraziden der allgemeinen Formel (III) |

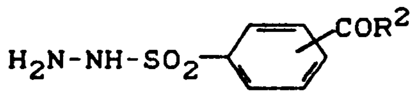 (III)

in welcher
$R^2$ die oben angegebene Bedeutung hat,
gegebenenfalls in Gegenwart eines Verdünnungsmittels umsetzt, oder wenn man

(b) Aminoguanidinotriazine der allgemeinen Formel (IV)

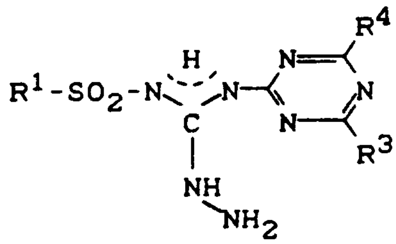 (IV)

in welcher
$R^1$, $R^3$ und $R^4$ die oben angegebenen Bedeutungen haben,
mit Sulfonsäurehalogeniden der allgemeinen Formel (V)

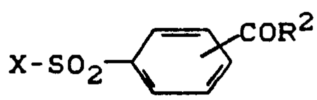 (V)

in welcher
$R^2$ die oben angegebene Bedeutung hat und
X für Halogen steht,
oder mit Sulfobenzoesäureanhydrid der Formel (VI)

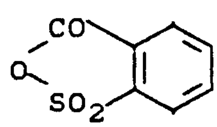 (VI)

gegebenenfalls in Gegenwart eines Verdünnungsmittels und gegebenenfalls in Gegenwart eines Säureakzeptors umsetzt, oder wenn man

(c) substituierte Arylsulfonylaminoguanidinotriazine der allgemeinen Formel (I), in welcher $R^2$ für gegebenenfalls substituiertes Alkoxy steht und $R^1$, $R^3$ und $R^4$ die oben angegebenen Bedeutungen haben, mit wäßrigen Alkalilaugen oder mit Ammoniak oder mit gegebenenfalls substituierten Alkylaminen gegebenenfalls in Gegenwart eines Verdünnungsmittels umsetzt.

Die neuen substituierten Arylsulfonylaminoguanidinotriazine der allgemeinen Formel (I) zeichnen sich durch starke herbizide Wirksamkeit aus.

Gegenstand der Erfindung sind vorzugsweise Verbindungen der Formel (I), in welcher
$R^1$ für den Rest

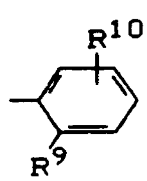

steht, worin

$R^9$ und $R^{10}$ gleich oder verschieden sind und für Wasserstoff, Fluor, Chlor, Brom, Iod, Cyano, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)-amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylaminocarbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthiooder $C_1$-$C_4$-Alkoxycarbonyl substituiert ist), $C_3$-$C_6$-Alkinyloxy, $C_3$-$C_6$-Alkinylthio oder für den Rest $-S(O)_p-R^{11}$ stehen, wobei

p für die Zahlen 1 oder 2 steht und

$R^{11}$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^{12}$ steht, wobei

$R^{12}$ für $C_1$-$C_{12}$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxycarbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzylhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^9$ und $R^{10}$ weiterhin für Phenyl oder Phenoxy, für Amino, $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest $-CO-R^{13}$ stehen, wobei

$R^{13}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegegebenenfalls durch Fluor und/oder Chlor substituiert sind),

$R^9$ und $R^{10}$ weiterhin für $C_1$-$C_4$-Alkylsulfonyloxy, Di-($C_1$-$C_4$-alkyl)-aminosulfonylamino, Thiazolyloxy oder für den Rest $-CH=N-R^{14}$ stehen, wobei

$R^{14}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

weiterhin

$R^1$ für den Rest

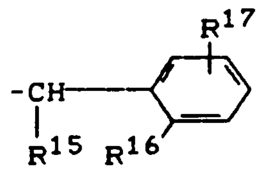

steht, worin

$R^{15}$ für Wasserstoff oder $C_1$-$C_4$-Alkyl steht,

$R^{16}$ und $R^{17}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/ oder Chlor substituiert ist), Carboxy, $C_1$-$C_4$-Alkoxy-carbonyl, $C_1$-$C_4$-Alkylsulfonyl oder Di-($C_1$-$C_4$-alkyl)-aminosulfonyl stehen; weiterhin

$R^1$ für den Rest

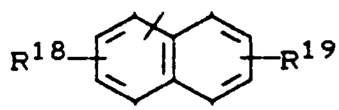

steht, worin

$R^{18}$ und $R^{19}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen; weiterhin

$R^1$ für den Rest

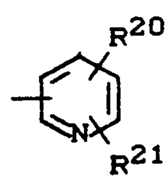

steht, worin

$R^{20}$ und $R^{21}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/ oder Chlor substituiert sind), sowie für Di-($C_1$-$C_4$-alkyl)-aminocarbonyl oder $C_1$-$C_4$-Alkoxy-carbonyl stehen; weiterhin

$R^1$ für den Rest

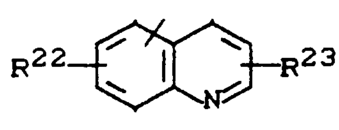

steht, worin

$R^{22}$ und $R^{23}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen; weiterhin

$R^1$ für den Rest

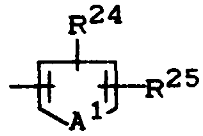

steht, worin

$R^{24}$ und $R^{25}$ gleich oder verschieden sind und für Wasserstoff, Fluor, Chlor, Brom, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-amino-sulfonyl oder $C_1$-$C_4$-Alkoxy-carbonyl stehen, und

$A^1$ für Sauerstoff, Schwefel oder die Gruppierung N-$Z^1$ steht, wobei

$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht; weiterhin

$R^1$ für den Rest

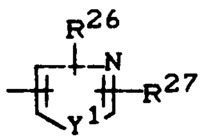

steht, worin

$R^{26}$ für Wasserstoff, $C_1$-$C_5$-Alkyl oder Halogen steht,

$R^{27}$ für Wasserstoff oder $C_1$-$C_5$-Alkyl steht und

$Y^1$ für Schwefel oder die Gruppierung N-$R^{28}$ steht, wobei

$R^{28}$ für Wasserstoff oder $C_1$-$C_5$-Alkyl steht, weiterhin

$R^1$ für den Rest

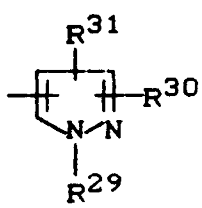

steht, worin

$R^{29}$ für Wasserstoff, $C_1$-$C_4$-Alkyl, Phenyl, Naphthyl oder (Iso)chinolinyl steht,

$R^{30}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist) oder $C_1$-$C_4$-Alkoxy-carbonyl steht und

$R^{31}$ für Wasserstoff, Halogen oder $C_1$-$C_4$-Alkyl steht,

weiterhin

$R^1$ für den Rest

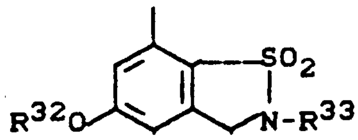

steht, worin

$R^{32}$ für $C_1$-$C_3$-Alkyl steht und

$R^{33}$ für $C_1$-$C_4$-Alkyl steht,

weiterhin

$R^1$ für den Rest

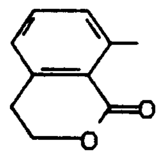

steht,

weiterhin

$R^2$ für Hydroxy, Amino oder für jeweils gegebenenfalls durch Fluor, Chlor, Methoxy oder Ethoxy substituiertes $C_1$-$C_4$-Alkoxy oder $C_1$-$C_4$-Alkylamino steht,

$R^3$ für Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino, Dimethylamino oder Diethylamino steht, und

$R^4$ für Fluor, Chlor, Brom, $C_1$-$C_4$-Alkyl, Cyclopropyl, $C_1$-$C_4$-Alkoxy, $C_1$-$C_4$-Alkylthio, $C_1$-$C_4$-Alkylamino, Dimethylamino oder Diethylamino steht,

wobei N'-(4,6-Dimethoxy-s-triazin-2-yl)-N''-(2-methoxycarbonyl-phenylsulfonylamino)-N'''-(2-brom-phenylsulfonyl)-guanidin, N'-(4,6-Dimethoxy-s-triazin-2-yl)-N''-(2-methoxycarbonyl-phenylsulfonylamino)-N'''-(2-phenyl-phenylsulfonyl)-guanidin, N'-(4-Methoxy-6-methyl-s-triazin-2-yl)-N''-(2-methoxycarbonyl-phenylsulfonylamino)-N'''-(2-phenyl-phenylsulfonyl)-guanidin, N'-(4,6-Dimethoxy-s-triazin-2-yl)-N''-(2-methoxycarbonyl-phenylsulfonylamino)-N'''-(2-methylthio-phenylsulfonyl)-guanidin und N-(4,6-Dimethoxy-s-triazin-2-yl)-N''-(2-methoxycarbonyl-phenylsulfonylamino)-N'''-(2-isopropoxycarbonyl-phenylsulfonyl)-guanidin ausgenommen sind (vgl. DE-P 3927770).

Gegenstand der Erfindung sind insbesondere Verbindungen der Formel (I), in welcher

$R^1$ für den Rest

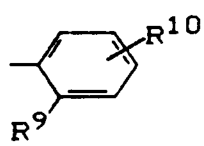

steht worin

$R^9$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy, Methoxycarbonyl, Ethoxycarbonyl, Propoxycarbonyl, Isopropoxycarbonyl, Chlorethoxycarbonyl, Methoxyethoxycarbonyl, n-, i- oder s-Butoxycarbonyl, n-, i- oder s-Pentoxycarbonyl steht und

$R^{10}$ für Wasserstoff, Fluor oder Chlor steht;

weiterhin

$R^1$ für den Rest

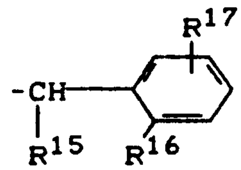

steht, worin

$R^{15}$ für Wasserstoff steht,

$R^{16}$ für Fluor, Chlor, Brom, Methyl, Methoxy, Ethoxy, Difluormethoxy, Trifluormethoxy, Methoxycarbonyl, Ethoxycarbonyl, Methylsulfonyl oder Dimethylaminosulfonyl steht und

$R^{17}$ für Wasserstoff, Fluor oder Chlor steht;

weiterhin

$R^1$ für den Rest

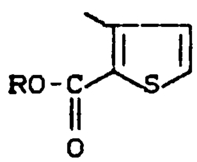

steht, worin

R für Methyl oder Ethyl steht, oder

$R^1$ für den Rest

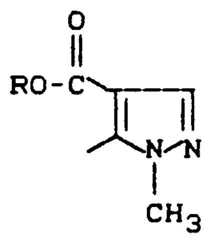

steht, worin

R für Methyl oder Ethyl steht,

$R^2$ für Hydroxy, Amino, Methoxy, Ethoxy, Propoxy, Isopropoxy, 2-Chlor-ethoxy, 2-Methoxy-ethoxy, 2-Ethoxy-ethoxy, Methylamino, Ethylamino, Propylamino oder Isopropylamino steht,

$R^3$ für Chlor, Methyl, Trifluormethyl, Methoxy, Ethoxy, Methylthio, Ethylthio, Methylamino, Ethylamino, Dimethylamino oder Diethylamino steht und

$R^4$ für Methyl, Ethyl, Cyclopropyl, Methoxy, Ethoxy, Propoxy, Isopropoxy, Methylthio, Ethylthio, Methylamino, Ethylamino, Dimethylamino oder Diethylamino steht,

wobei N'-(4,6-Dimethoxy-s-triazin-2-yl)-N''-(2-methoxycarbonyl-phenylsulfonylamino)-N'''-(2-brom-phenylsulfonyl)-guanidin, N'-(4,6-Dimethoxy-s-triazin-2-yl)-N''-(2-methoxycarbonyl-phenylsulfonylamino)-N'''-(2-phenyl-phenylsulfonyl)-guanidin, N'-(4-Methoxy-6-methyl-s-triazin-2-yl)-N''-(2-methoxycarbonyl-phenylsulfonylamino)-N'''-(2-phenyl-phenylsulfonyl)-guanidin, N'-(4,6-Dimethoxy-s-triazin-2-yl)-N''-(2-methoxycarbonyl-phenylsulfonylamino)-N'''-(2-methylthio-phenylsulfonyl)-guanidin und N-(4,6-Dimethoxy-s-triazin-2-yl)-N''-(2-methoxycarbonyl-phenylsulfonylamino)-N'''-(2-isopropoxycarbonyl-phenylsulfonyl)-guanidin ausgenommen sind (vgl. DE-P 3927770).

Ganz besonders bevorzugt sind diejenigen Verbindungen der Formel (I) bei welchen die Gruppierung $-COR^2$ sich in ortho-Position zur $SO_2$-Gruppe befindet und $R^1$, $R^2$, $R^3$ und $R^4$ die oben als insbesondere bevorzugt angegebenen Bedeutungen haben, wobei die vorausgehend angegebenen Verbindungen ausgenommen sind.

Verwendet man beispielsweise N'-(4,6-Dimethoxy-s-triazin-2-yl)-N''-methoxy-N'',N'''-bis-(2-fluor-phenylsulfonyl)-guanidin und 2-Methoxycarbonyl-benzolsulfonsäurehydrazid als Ausgangsstoffe, so kann der Reaktionsablauf beim erfindungsgemäßen Verfahren (a) durch das folgende Formelschema skiziert werden:

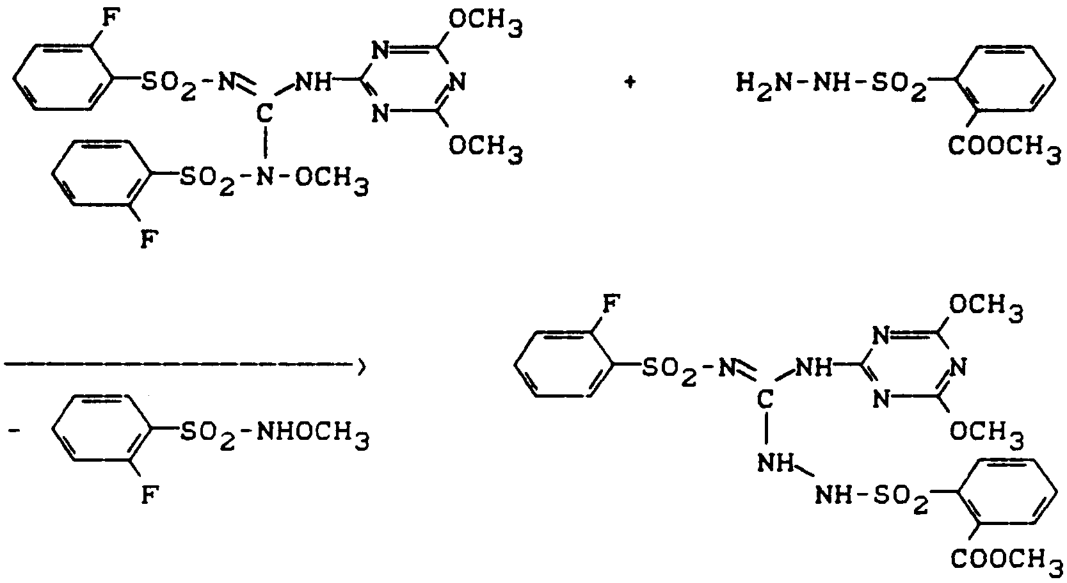

Verwendet man beispielsweise N'-(4,6-Dimethoxy-s-triazin-2-yl)-N''-amino-N'''-(2-difluormethoxy-phenylsulfonyl)-guanidin und 2-Ethoxycarbonylbenzolsulfonsäurechlorid als Ausgangsstoffe, so kann der Reaktionsablauf beim erfindungsgemäßen Verfahren (b) durch das folgende Formelschema skizziert werden:

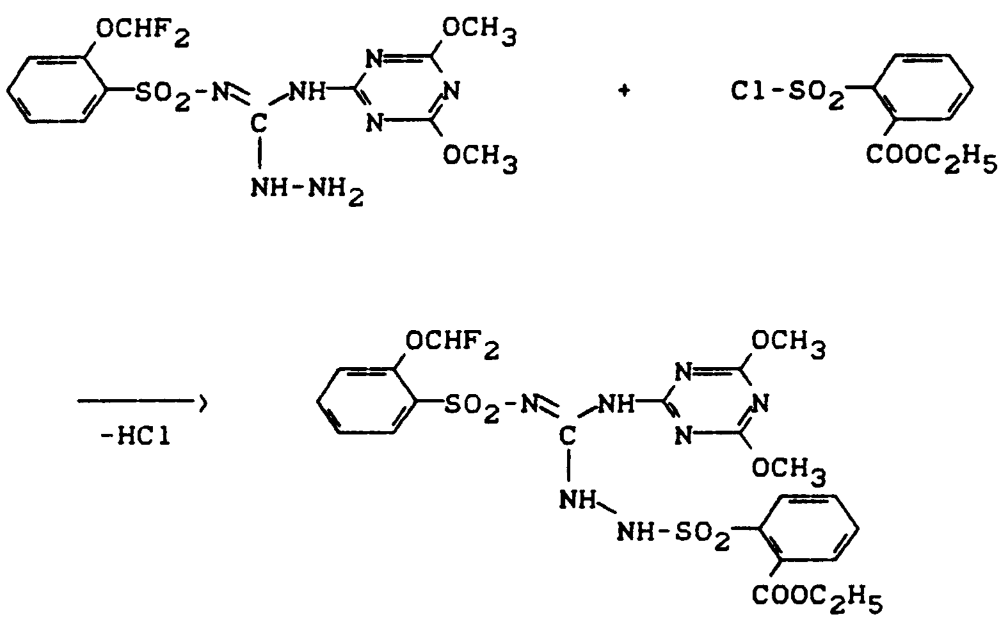


Verwendet man beispielsweise N'-(4-Methoxy-6-methyl-s-triazin-2-yl)-N''-(2-methoxycarbonyl-phenylsulfonylamino)-N'''-(2-methoxycarbonyl-phenylsulfonyl)-guanidin und Ammoniak als Ausgangsstoffe, so kann der Reaktionsablauf beim erfindungsgemäßen Verfahren (c) durch das folgende Formelschema skizziert werden:

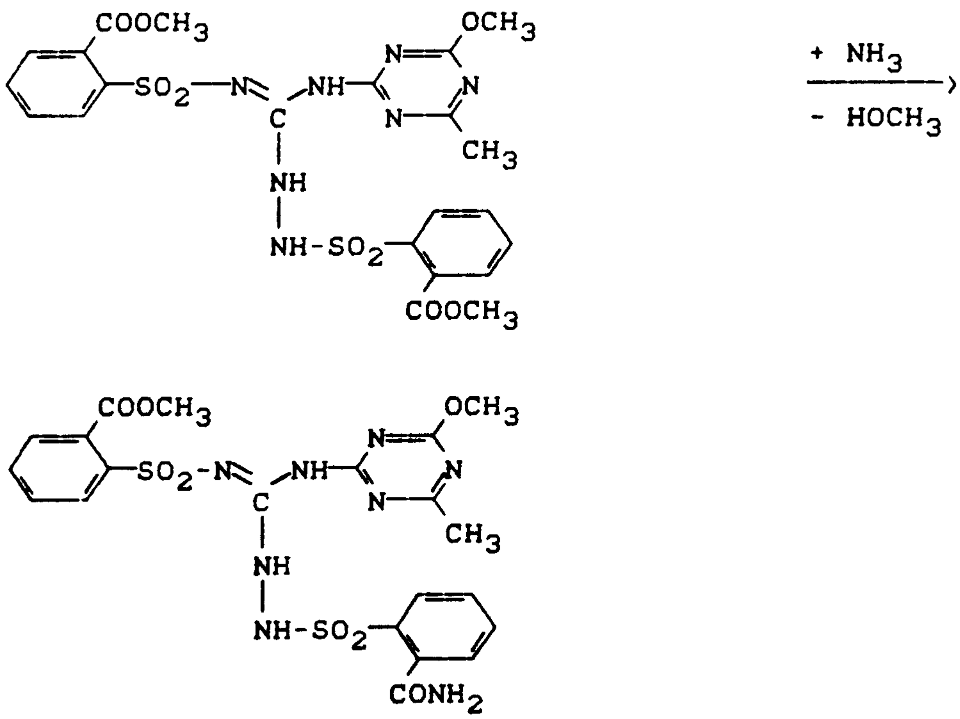


Die beim erfindungsgemäßen Verfahren (a) als Ausgangsstoffe zu verwendenden Sulfonylverbindungen sind durch die Formel (II) allgemein definiert. In Formel (II) haben $R^1$, $R^3$ und $R^4$ vorzugsweise bzw. insbesondere diejenigen Bedeutungen, die bereits oben im Rahmen der Beschreibung der erfindungsgemäßen Verbindungen der Formel (I) als bevorzugt bzw. als insbesondere bevorzugt für $R^1$, $R^3$ und $R^4$ angegeben wurden und

$R^5$ steht vorzugsweise für eine der nachstehend angegebenen Abgangsgruppen

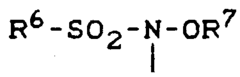

oder $-Q-R^8$ , worin

- $R^6$ die oben für $R^1$ vorzugsweise angegebene Bedeutung hat, aber nicht in jedem Einzelfall mit $R^1$ identisch sein muß,
- $R^7$ für $C_1$-$C_4$-Alkyl, $C_3$-$C_4$-Alkenyl oder Benzyl steht,
- $R^8$ für $C_1$-$C_4$-Alkyl, Benzyl oder Phenyl steht und
- Q für Sauerstoff oder Schwefel steht.

Insbesondere steht $R^5$ für die Gruppierung

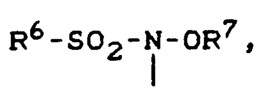

worin

- $R^6$ die oben für $R^1$ als insbesondere bevorzugt angegebene Bedeutung hat, aber nicht in jedem Einzelfall mit $R^1$ identisch sein muß und
- $R^7$ für Methyl steht,

oder $R^5$ steht für die Gruppierung

$-Q-R^8$, worin

- $R^8$ für Methyl oder Phenyl steht und
- Q für Sauerstoff oder Schwefel steht,

Die Ausgangsstoffe der Formel (II) sind bekannt und/oder können nach an sich bekannten Verfahren

hergestellt werden (vgl. EP-A 121 082, EP-A 172 957, EP-A 173 321, EP-A 173 956, EP-A 224 078, EP-A 5 986 und EP-A 24 215).

Die beim erfindungsgemäßen Verfahren weiter als Ausgangsstoffe zu verwendenden Sulfonsäurehydrazide sind durch die Formel (III) allgemein definiert. In Formel (III) hat $R^2$ vorzugsweise bzw. insbesondere diejenige Bedeutung, die bereits oben im Rahmen der Beschreibung der erfindungsgemäßen Vebindungen der Formel (I) als bevorzugt bzw. als insbesondere bevorzugt für $R^2$ angegeben wurde.

Als Beispiele für die Verbindungen der Formel (III) seien genannt:

2-Methoxycarbonyl-, 4-Methoxycarbonyl-, 2-Ethoxycarbonyl- und 4-Ethoxycarbonyl-benzolsulfonsäurehydrazid.

Die Ausgangsstoffe der Formel (III) sind bekannt und/oder können nach an sich bekannten Verfahren hergestellt werden (vgl. Org. Synth. 40 (1960), 93 - 95; EP-A 302378; Egypt. J. Pharm. Sci 22(1981), 207-221 - zit. in Chem. Abstracts 100, 191704y).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei vorzugsweise Wasser und/oder polare organische Lösungsmittel, wie Methanol, Ethanol, Isopropanol, Butanol, Isobutanol, sec-Butanol, tert-Butanol, Glycoldimethylether, Diglycoldimethylether, Tetrahydrofuran, Dioxan, Essigsäuremethylester, Essigsäureethylester, Acetonitril, Propionitril, Dimethylformamid, Dimethylacetamid, N-Methylpyrrolidon, Dimethylsulfoxid und Tetramethylensulfon, in Betracht.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens (a) setzt man je Mol Sulfonylverbindung der Formel (II) im allgemeinen zwischen 1 und 5 Mol, vorzugsweise zwischen 1 und 3 Mol, Sulfonsäurehydrazid der Formel (III) ein.

Im allgemeinen werden die Reaktionskomponenten bei Raumtemperatur oder unter Eiskühlung zusammen gegeben und das Reaktionsgemisch wird, gegebenenfalls bei erhöhter Temperatur, bis zum Ende der Umsetzung gerührt. Die Produkte der Formel (I) fallen im allgemeinen nach dem Abkühlen kristallin an und können durch Absaugen isoliert werden.

Die beim erfindungsgemäßen Verfahren (b) zur Herstellung von Verbindungen der Formel (I) als Ausgangsstoffe zu verwendenden Aminoguanidinotriazine sind durch die Formel (IV) allgemein definiert.

In Formel (IV) haben $R^1$, $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^3$ und $R^4$ angegeben wurden.

Die Ausgangsstoffe der Formel (IV) sind bekannt und/oder können nach an sich bekannten Verfahren hergestellt werden (vgl. EP-A 224078, US-P 4725303, EP-A 343462).

Man erhält die Aminoguanidinotriazine der Formel (IV), wenn man Sulfonylverbindungen der allgemeinen Formel (II) -oben - analog zum erfindungsgemäßen Verfahren (a) mit Hydrazin oder einem Hydrazin-Wasser-Addukt ("Hydrazin-Hydrat") gegebenenfalls in Gegenwart eines Verdünnungsmittels, wie z.B. Methylenchlorid, Methanol oder Ethanol, und gegebenenfalls in Gegenwart eines Trockenmittels, wie z.B. Natriumsulfat, bei Temperaturen zwischen -20 °C und +80 °C, vorzugsweise zwischen 0 °C und 50 °C, umsetzt.

Die beim erfindungsgemäßen Verfahren (b) weiter als Ausgangsstoffe zu verwendenden Sulfonsäurehalogenide sind durch die Formel (V) allgemein definiert.

In Formel (V) hat $R^2$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^2$ angegeben wurde und X steht vorzugsweise für Fluor, Chlor oder Brom, insbesondere für Chlor.

Als Beispiele für die Ausgangsstoffe der Formel (V) seien genannt:

2-Methoxycarbonyl- und 2-Ethoxycarbonyl- sowie 4-Methoxycarbonyl- und 4-Ethoxycarbonyl-benzolsulfonsäurechlorid.

Die Ausgangsstoffe der Formel (V) sind - wie auch die Ausgangsverbindung der Formel (VI) bekannt und/oder können nach an sich bekannten Verfahren hergestellt werden (vgl. EP-A 173320 und EP-A 173321).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2, 2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen -100 °C und +100 °C, vorzugsweise bei Temperaturen zwischen -70 °C und +70 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel, gegebenenfalls in Gegenwart eines Säureakzeptors, durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die beim erfindungsgemäßen Verfahren (c) als Ausgangsstoffe zu verwendenden substituierten Arylsulfonylaminoguanidinotriazine sind durch die Formel (I) mit der Maßgabe, daß darin $R^2$ für gegebenenfalls substituiertes Alkoxy steht, allgemein definiert. In diesem Fall haben $R^1$, $R^3$ und $R^4$ vorzugsweise bzw. insbesondere diejenigen Bedeutungen, die bereits oben im Rahmen der Beschreibung der erfindungsgemäßen Verbindungen der Formel (I) als bevorzugt bzw. als besonders bevorzugt für $R^1$, $R^3$ und $R^4$ angegeben wurden und $R^2$ steht vorzugsweise für gegebenenfalls durch Fluor, Chlor, Methoxy oder Ethoxy substituiertes $C_1$-$C_4$-Alkoxy, insbesondere für Methoxy, Ethoxy, Propoxy, Isopropoxy, 2-Chlor-ethoxy, 2-Methoxy-ethoxy oder 2-Ethoxy-ethoxy.

Die beim erfindungsgemäßen Verfahren (c) als Ausgangsstoffe einzusetzenden Verbindungen der Formel (I) sind erfindungsgemäße, neue Verbindungen; sie können nach den erfindungsgemäßen Verfahren (a) oder (b) hergestellt werden.

Wäßrige Alkalilaugen, die beim erfindungsgemäßen Verfahren (c) eingesetzt werden können, sind Lösungen von Alkalimetallhydroxiden, wie z.B. von Lithiumhydroxid, Natriumhydroxid oder Kaliumhydroxid, in Wasser. Vorzugsweise wird wäßrige Natronlauge beim erfindungsgemäßen Verfahren (c) eingesetzt.

Ammoniak kann beim erfindungsgemäßen Verfahren (c) als Gas oder in Lösung, vorzugsweise in wäßriger Lösung eingesetzt werden.

Gegebenenfalls substituierte Alkylamine, welche beim erfindungsgemäßen Verfahren (c) eingesetzt werden können, sind vorzugsweise Alkylamine mit 1 bis 4 Kohlenstoffatomen, welche gegebenenfalls durch Fluor, Chlor, Methoxy oder Ethoxy substituiert sind, insbesondere Methylamin, Ethylamin, Propylamin und Isopropylamin.

Das erfindungsgemäße Verfahren (c) wird gegebenenfalls unter Verwendung von Verdünnungsmitteln durchgeführt. Als Verdünnungsmittel kommen dabei neben Wasser und Alkoholen, wie z.B. Methanol, Ethanol und Isopropanol praktisch alle inerten organischen Lösungsmittel in Frage. Hierzu gehören Vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Die Reaktionstemperaturen können bei dem erfindungsgemäßen Verfahren (c) in einem größeren Bereich variiert werden. Im allgemeinen arbeitet man bei Temperaturen zwischen 0 °C und 120 °C, vorzugsweise bei Temperaturen zwischen 20 °C und 100 °C.

Das erfindungsgemäße Verfahren (c) wird im allgemeinen unter Normaldruck durchgeführt. Es ist

jedoch auch möglich, unter erhöhtem oder vermindertem Druck zu arbeiten.

Zur Durchführung des erfindungsgemäßen Verfahrens (c) setzt man auf 1 Mol Ausgangsverbindung der Formel (I) im allgemeinen zwischen 1 und 10 Mol, vorzugsweise zwischen 1 und 5 Mol, Alkalihydroxid, Ammoniak oder Amin ein.

Im allgemeinen wird die Ausgangsverbindung der Formel (I) bei Raumtemperatur, gegebenenfalls in einem Verdünnungsmittel, vorgelegt und die wäßrige Alkalilauge, das Ammoniak oder das Amin wird, gegebenenfalls bei erhöhter Temperatur, zudosiert. Das Reaktionsgemisch wird bis zum Ende der Umsetzung gerührt und nach üblichen Methoden aufgearbeitet (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage:
z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit Bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxy]-phenoxy}-propansäure, deren Methyl-oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 4-Ethylamino-2-t-butylamino-6-methylthio-s-triazin(TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele:

### Beispiel 1

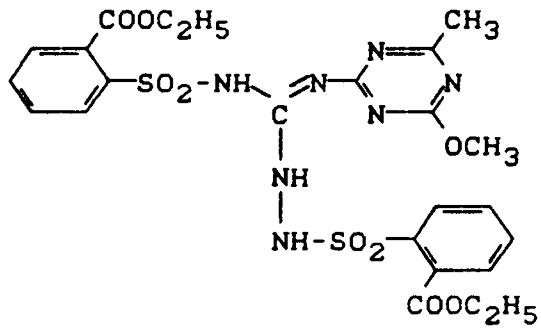

(Verfahren (b))

Eine Mischung aus 7,5 g (18,3 mmol) N'-(4-Methoxy-6-methyl-s-triazin-2-yl)-N''-amino-N'''-(2-ethoxycarbonylphenylsulfonyl)-guanidin, 2,28 g (20,3 mmol) Diazabicyclo-[2,2,2]-octan (DABCO) und 100 ml Methylenchlorid wird auf -70 ° C abgekühlt und dazu wird unter Rühren eine Lösung von 4,53 g (18,3 mmol) 2-Ethoxycarbonyl-benzolsulfonsäurechlorid in 20 ml Methylenchlorid tropfenweise gegeben. Nach Entfernen des Kühlbades wird das Reaktionsgemisch weitere 15 Stunden (am Ende bei 20 ° C) gerührt. Anschließend wird mit 100 ml Methylenchlorid verdünnt und zweimal mit je ca. 200 ml Wasser gewaschen. Die organische Phase wird mit Magnesiumsulfat getrocknet und filtriert. Vom Filtrat wird das Lösungsmittel im Wasserstrahlvakuum abdestilliert, der Rückstand mit Essigester zur Kristallisation gebracht und das kristalline Produkt durch Absaugen isoliert.

Man erhält 6,3 g einer ersten Fraktion von N'-(4-Methoxy-6-methyl-s-triazin-2-yl)-N''-(2-ethoxycarbonylphenylsulfonylamino)-N'''-(2-ethoxycarbonyl-phenylsulfonyl)-guanidin. Die Mutterlauge wird eingeengt und der hierbei verbleibende Rückstand mit Ethanol umkristallisiert, wobei eine zweite Produktfraktion (1,55 g) anfällt. Gesamt-Ausbeute: 7,85 g (69% der Theorie); Schmelzpunkt: 165 ° C.

Analog Beispiel 1 und entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

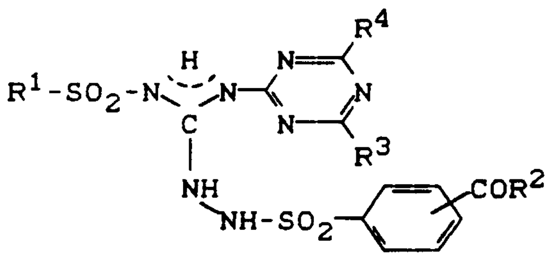

(I)

**Tabelle 1:** Beispiele für die Verbindungen der Formel (I)

| Bsp.-Nr. | $R^1$ | Position $-COR^2$ | $R^3$ | $R^4$ | Schmelzpunkt (°C) |
|---|---|---|---|---|---|
| 2 | 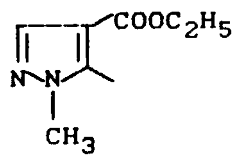 | (2-)$COOCH_3$ | $OCH_3$ | $OCH_3$ | 162 |
| 3 | 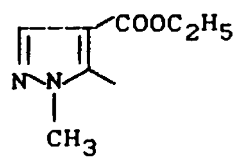 | (2-)$COOCH(CH_3)_2$ | $OCH_3$ | $OCH_3$ | 134 |
| 4 | 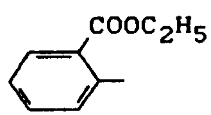 | (2-)$COOCH_3$ | $CH_3$ | $OCH_3$ | 149 |
| 5 | 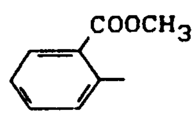 | (2-)$COOCH_3$ | $OCH_3$ | $OCH_3$ | 97 |
| 6 | 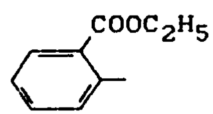 | (2-)$COOCH_3$ | $OCH_3$ | $OCH_3$ | 145 |
| 7 | 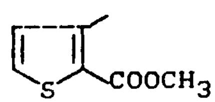 | (2-)$COOCH_3$ | $CH_3$ | $OCH_3$ | 132 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 8 | 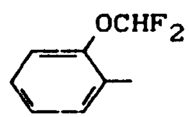 | $(2-)COOCH_3$ | $OCH_3$ | $OCH_3$ | 163 |
| 9 | 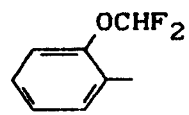 | $(2-)COOC_2H_5$ | $OCH_3$ | $OCH_3$ | 162 |
| 10 | 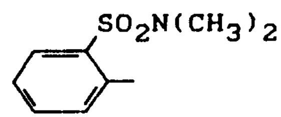 | $(2-)COOCH_3$ | $CH_3$ | $OCH_3$ | 189 |
| 11 | 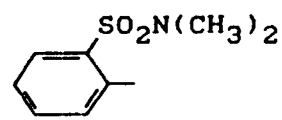 | $(2-)COOC_2H_5$ | $CH_3$ | $OCH_3$ | 176 |
| 12 | 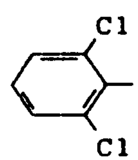 | $(2-)COOCH_3$ | $CH_3$ | $OCH_3$ | 184 |

**Tabelle 1** - Fortsetzung

| Bsp.-Nr. | $R^1$ | Position $-COR^2$ | $R^3$ | $R^4$ | Schmelzpunkt (°C) |
|---|---|---|---|---|---|
| 13 | 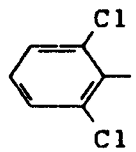 | (2-)$COOC_2H_5$ | $CH_3$ | $OCH_3$ | 126 |
| 14 | 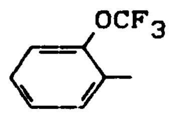 | (2-)$COOC_2H_5$ | $OCH_3$ | $OCH_3$ | 84 |
| 15 | 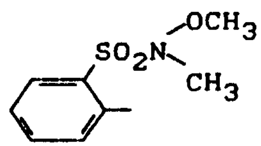 | (2-)$COOCH_3$ | $CH_3$ | $OCH_3$ | 192 |
| 16 | 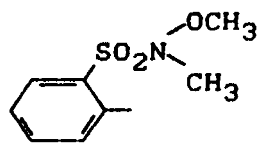 | (2-)$COOC_2H_5$ | $CH_3$ | $OCH_3$ | 167 |
| 17 | 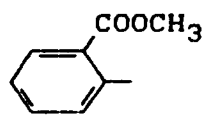 | (2-)$COOC_2H_5$ | $OCH_3$ | $OCH_3$ | 125 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 18 | 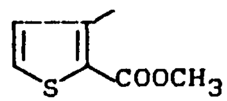 | (2-)$COOCH_3$ | $OCH_3$ | $OCH_3$ | 140 |
| 19 | 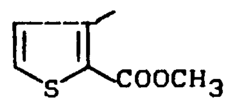 | (2-)$COOC_2H_5$ | $OCH_3$ | $OCH_3$ | 150 |
| 20 | 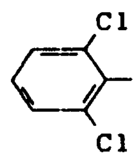 | (2-)$COOCH_3$ | $OCH_3$ | $OCH_3$ | 197 |
| 21 | 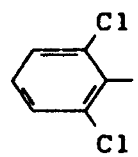 | (2-)$COOC_2H_5$ | $OCH_3$ | $OCH_3$ | 190 |
| 22 | 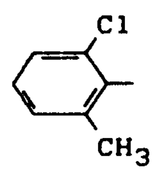 | (2-)$COOCH_3$ | $OCH_3$ | $OCH_3$ | 177 |

**Tabelle 1** - Fortsetzung

| Bsp.-Nr. | $R^1$ | Position $-COR^2$ | $R^3$ | $R^4$ | Schmelzpunkt (°C) |
|---|---|---|---|---|---|
| 23 | 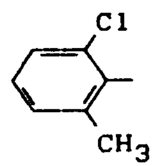 | $(2-)COOC_2H_5$ | $OCH_3$ | $OCH_3$ | 137 |
| 24 | 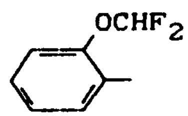 | $(2-)COOCH_3$ | $CH_3$ | $OCH_3$ | 167 |
| 25 | 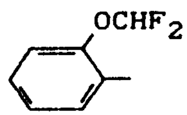 | $(2-)COOC_2H_5$ | $CH_3$ | $OCH_3$ | 160 |
| 26 | 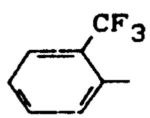 | $(2-)COOCH_3$ | $CH_3$ | $OCH_3$ | 149 |
| 27 | 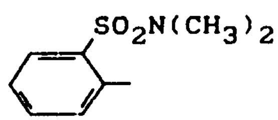 | $(2-)COOCH_3$ | $OCH_3$ | $OCH_3$ | 173 |
| 28 | 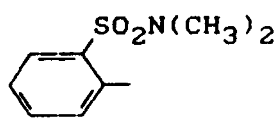 | $(2-)COOC_2H_5$ | $OCH_3$ | $OCH_3$ | 182 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 29 | 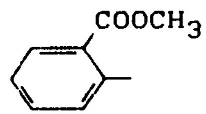 | (2-)$COOCH_3$ | $CH_3$ | $OCH_3$ | 149 |
| 30 | 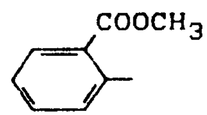 | (2-)$COOC_2H_5$ | $CH_3$ | $OCH_3$ | 142 |
| 31 | 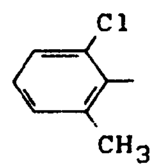 | (2-)$COOCH_3$ | $CH_3$ | $OCH_3$ | 131 |
| 32 | 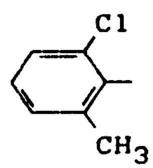 | (2-)$COOC_2H_5$ | $CH_3$ | $OCH_3$ | 119 |
| 33 | 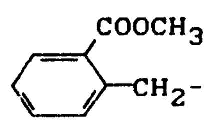 | (2-)$COOCH_3$ | $CH_3$ | $OCH_3$ | 159 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 34 | 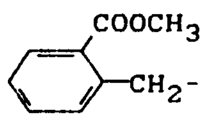 | (2-)$COOC_2H_5$ | $CH_3$ | $OCH_3$ | 172 |
| 35 | 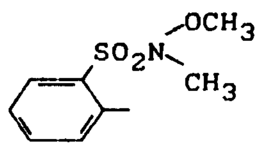 | (2-)$COOCH_3$ | $OCH_3$ | $OCH_3$ | 186 |
| 36 | 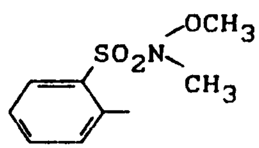 | (2-)$COOC_2H_5$ | $OCH_3$ | $OCH_3$ | 176 |
| 37 | 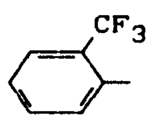 | (2-)$COOCH_3$ | $OCH_3$ | $OCH_3$ | 116 |
| 38 | 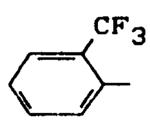 | (2-)$COOC_2H_5$ | $OCH_3$ | $OCH_3$ | 88 |

**Tabelle 1** - Fortsetzung

| Bsp.-Nr. | $R^1$ | Position $-COR^2$ | $R^3$ | $R^4$ | Schmelzpunkt (°C) |
|---|---|---|---|---|---|
| 39 | 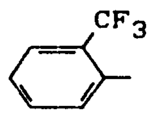 | (2-)$COOC_2H_5$ | $CH_3$ | $OCH_3$ | 91 |
| 40 | 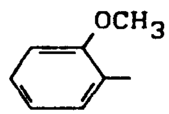 | (2-)$COOCH_3$ | $CH_3$ | $OCH_3$ | 119 |
| 41 | 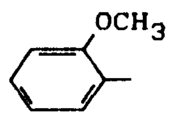 | (2-)$COOC_2H_5$ | $CH_3$ | $OCH_3$ | 87 |
| 42 | 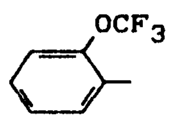 | (2-)$COOCH_3$ | $CH_3$ | $OCH_3$ | 82 |
| 43 | 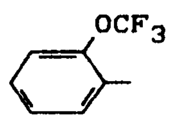 | (2-)$COOCH_3$ | $OCH_3$ | $OCH_3$ | 93 |
| 44 | 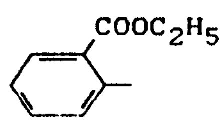 | (2-)$COOC_2H_5$ | $OCH_3$ | $OCH_3$ | 158 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 45 | 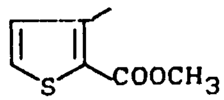 | (2-)$COOC_3H_7n$ | $CH_3$ | $OCH_3$ | 95 |
| 46 | 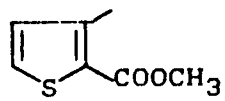 | (2-)$COOCH(CH_3)_2$ | $CH_3$ | $OCH_3$ | 104 |
| 47 | 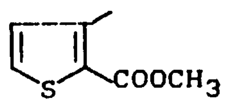 | (2-)$COOCH_2CH_2OCH_3$ | $CH_3$ | $OCH_3$ | 105 |
| 48 | 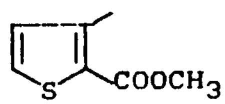 | (2-)-$COOCH_2CH_2Cl$ | $CH_3$ | $OCH_3$ | 104 |
| 49 | 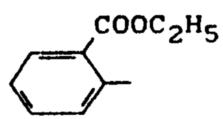 | (2-)$COOC_3H_7n$ | $OCH_3$ | $OCH_3$ | 80 |
| 50 | 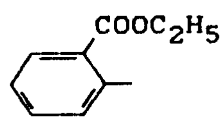 | (2-)$COOCH(CH_3)_2$ | $OCH_3$ | $OCH_3$ | 80 |
| 51 | 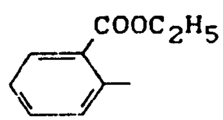 | (2-)$COOCH_2CH_2OCH_3$ | $OCH_3$ | $OCH_3$ | 84 |

**Tabelle 1** - Fortsetzung

| Bsp.-Nr. | $R^1$ | Position $-COR^2$ | $R^3$ | $R^4$ | Schmelzpunkt (°C) |
|---|---|---|---|---|---|
| 52 | 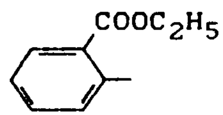 | (2-)$COOCH_2CH_2Cl$ | $OCH_3$ | $OCH_3$ | 84 |
| 53 | 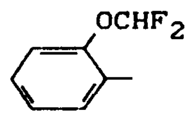 | (2-)$COOCH_2CH_2OCH_3$ | $OCH_3$ | $OCH_3$ | 160 |
| 54 | 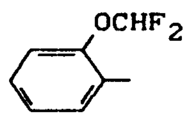 | (2-)$COOCH(CH_3)_2$ | $OCH_3$ | $OCH_3$ | 87 |
| 55 | 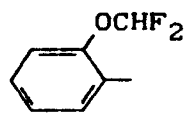 | (2-)$COOC_3H_7n$ | $OCH_3$ | $OCH_3$ | 134 |
| 56 | 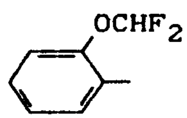 | (2-)$COOCH_2CH_2Cl$ | $OCH_3$ | $OCH_3$ | 93 |
| 57 | 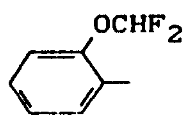 | (2-)$COOC_3H_7n$ | $CH_3$ | $OCH_3$ | 87 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 58 | 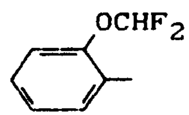 | (2-)$COOCH(CH_3)_2$ | $CH_3$ | $OCH_3$ | 95 |
| 59 | 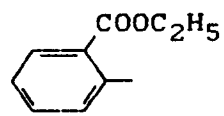 | (2-)$COOC_3H_7n$ | $CH_3$ | $OCH_3$ | 120 |
| 60 | 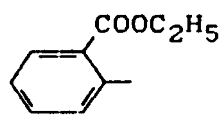 | (2-)$COOCH(CH_3)_2$ | $CH_3$ | $OCH_3$ | 81 |
| 61 | 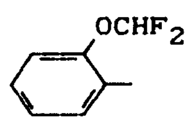 | (2-)$COOCH_2CH_2OCH_3$ | $CH_3$ | $OCH_3$ | 82 |
| 62 | 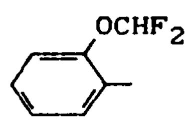 | (2-)$COOCH_2CH_2Cl$ | $CH_3$ | $OCH_3$ | 89 |
| 63 | 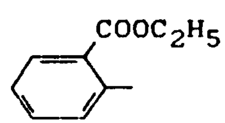 | (2-)$COOCH_2CH_2OCH_3$ | $CH_3$ | $OCH_3$ | 84 |

<u>**Tabelle 1**</u> - **Fortsetzung**

| Bsp.-Nr. | $R^1$ | Position $-COR^2$ | $R^3$ | $R^4$ | Schmelzpunkt (°C) |
|---|---|---|---|---|---|
| 64 | 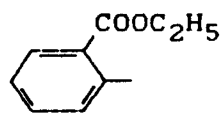 | (2-)$COOCH_2CH_2Cl$ | $CH_3$ | $OCH_3$ | 116 |
| 65 | 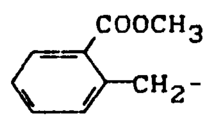 | (2-)$COOCH_3$ | $OCH_3$ | $OCH_3$ | 95 |
| 66 | 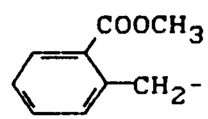 | (2-)$COOC_2H_5$ | $OCH_3$ | $OCH_3$ | 86 |
| 67 | 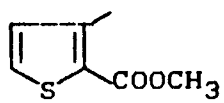 | (2-)$COOC_2H_5$ | $CH_3$ | $OCH_3$ | 98 |
| 68 | 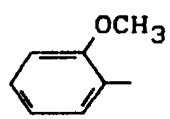 | (2-)$COOCH_3$ | $OCH_3$ | $OCH_3$ | 109 |
| 69 | 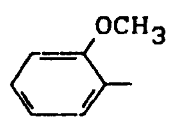 | (2-)$COOC_2H_5$ | $OCH_3$ | $OCH_3$ | 105 |

**Tabelle 1** - Fortsetzung

| Bsp.-Nr. | $R^1$ | Position $-COR^2$ | $R^3$ | $R^4$ | Schmelzpunkt (°C) |
|---|---|---|---|---|---|
| 70 | 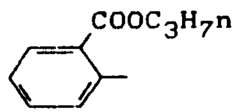 | (2-)$COOCH_3$ | $OCH_3$ | $OCH_3$ | 83 |
| 71 | 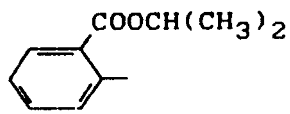 | (2-)$COOCH_3$ | $OCH_3$ | $OCH_3$ | 93 |
| 72 | 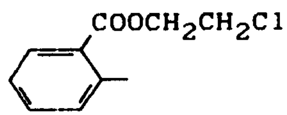 | (2-)$COOCH_3$ | $OCH_3$ | $OCH_3$ | 95 |
| 73 | 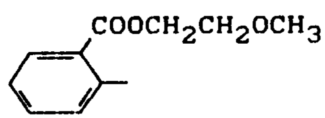 | (2-)$COOCH_3$ | $OCH_3$ | $OCH_3$ | 80 |
| 74 | 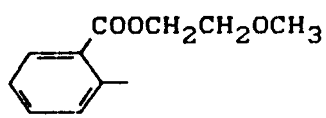 | (2-)$COOC_2H_5$ | $OCH_3$ | $OCH_3$ | 123 |
| 75 | 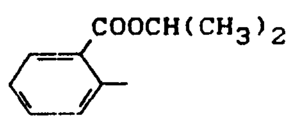 | (2-)$COOC_2H_5$ | $OCH_3$ | $OCH_3$ | 87 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 76 | 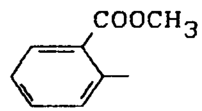 | (2-)$COOC_3H_7n$ | $OCH_3$ | $OCH_3$ | 93 |
| 77 | 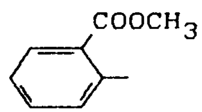 | (2-)$COOCH_2CH_2Cl$ | $OCH_3$ | $OCH_3$ | 95 |
| 78 | 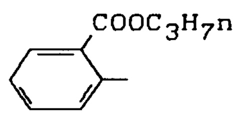 | (2-)$COOCH_3$ | $CH_3$ | $OCH_3$ | 91 |
| 79 | 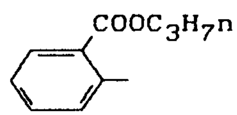 | (2-)$COOC_2H_5$ | $CH_3$ | $OCH_3$ | 86 |
| 80 | 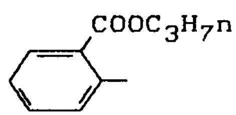 | (2-)$COOC_3H_7n$ | $CH_3$ | $OCH_3$ | 160 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 81 | 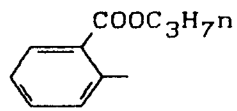 | (2-)$COOCH_2CH_2Cl$ | $CH_3$ | $OCH_3$ | 86 |
| 82 | 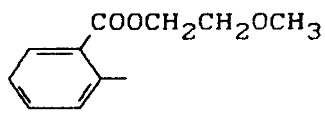 | (2-)$COOCH_3$ | $CH_3$ | $OCH_3$ | 86 |
| 83 | 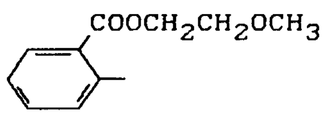 | (2-)$COOC_2H_5$ | $CH_3$ | $OCH_3$ | 86 |
| 84 | 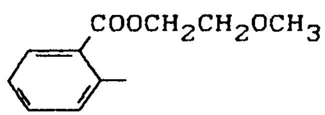 | (2-)$COOC_3H_7n$ | $CH_3$ | $OCH_3$ | 90 |
| 85 | 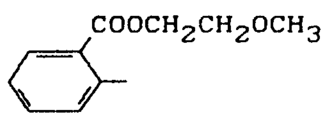 | (2-)$COOCH_2CH_2Cl$ | $CH_3$ | $OCH_3$ | 94 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 86 | 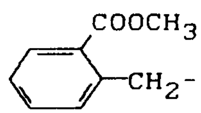 | (2-)$COOC_3H_7n$ | $OCH_3$ | $OCH_3$ | 169 |
| 87 | 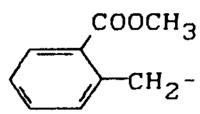 | (2-)$COOCH(CH_3)_2$ | $OCH_3$ | $OCH_3$ | 89 |
| 88 | 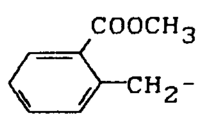 | (2-)$COOCH_2CH_2Cl$ | $OCH_3$ | $OCH_3$ | 153 |
| 89 | 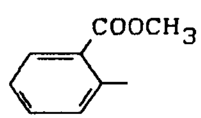 | (2-)$COOCH_3$ | $CH_3$ | $SCH_3$ | 94 |
| 90 | 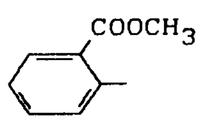 | (2-)$COOC_2H_5$ | $CH_3$ | $SCH_3$ | 94 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 91 | 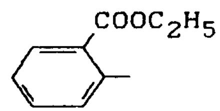 | (2-)$COOCH_3$ | $OCH_3$ | 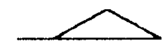 | 135 |
| 92 | 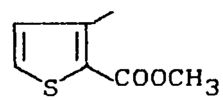 | (2-)$COOCH_3$ | $OCH_3$ | 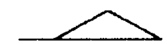 | 98 |
| 93 | 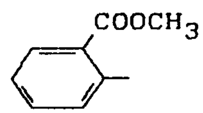 | (2-)$COOC_3H_7n$ | $CH_3$ | $OCH_3$ | 92 |
| 94 | 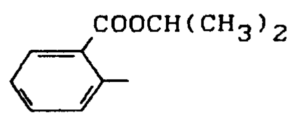 | (2-)$COOCH_3$ | $CH_3$ | $OCH_3$ | 108 |
| 95 | 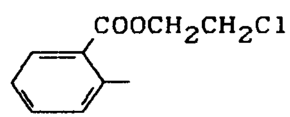 | (2-)$COOCH_3$ | $CH_3$ | $OCH_3$ | 100 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 96 | 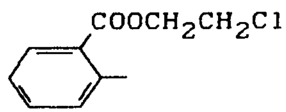 | (2-)$COOC_2H_5$ | $CH_3$ | $OCH_3$ | 86 |
| 97 | 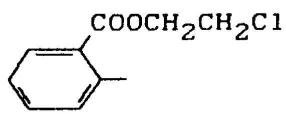 | (2-)$COOC_3H_7n$ | $CH_3$ | $OCH_3$ | 80 |
| 98 | 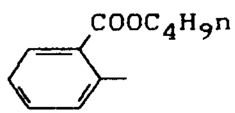 | (2-)$COOCH_3$ | $CH_3$ | $OCH_3$ | 87 |
| 99 | 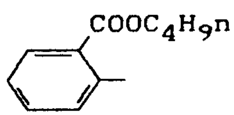 | (2-)$COOC_2H_5$ | $CH_3$ | $OCH_3$ | 75 |
| 100 | 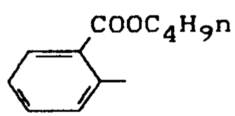 | (2-)$COOC_3H_7n$ | $CH_3$ | $OCH_3$ | 78 |

Tabelle 1 - Fortsetzung

| Bsp.-Nr. | $R^1$ | Position $-COR^2$ | $R^3$ | $R^4$ | Schmelz-punkt (°C) |
|---|---|---|---|---|---|
| 101 | 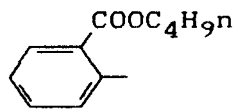 | (2-)$COOCH(CH_3)_2$ | $CH_3$ | $OCH_3$ | 78 |
| 102 | 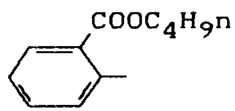 | (2-)$COOCH_2CH_2OCH_3$ | $CH_3$ | $OCH_3$ | 79 |
| 103 | 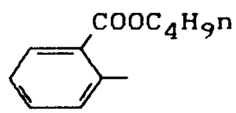 | (2-)$COOCH_3$ | $OCH_3$ | $OCH_3$ | 83 |
| 104 | 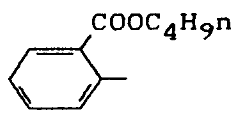 | (2-)$COOC_2H_5$ | $OCH_3$ | $OCH_3$ | 82 |
| 105 | 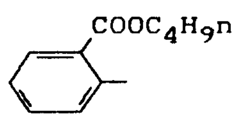 | (2-)$COOC_3H_7n$ | $OCH_3$ | $OCH_3$ | 136 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 106 | 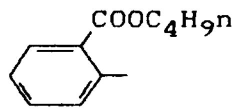 | (2-)$COOCH(CH_3)_2$ | $OCH_3$ | $OCH_3$ | 76 |
| 107 | 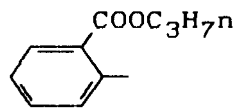 | (2-)$COOCH(CH_3)_2$ | $CH_3$ | $OCH_3$ | 80 |
| 108 | 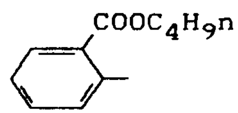 | (2-)$COOCH_2CH_2Cl$ | $OCH_3$ | $OCH_3$ | 79 |
| 109 | 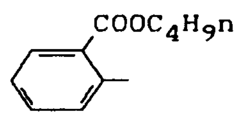 | (2-)$COOCH_2CH_2OCH_3$ | $OCH_3$ | $OCH_3$ | 75 |
| 110 | 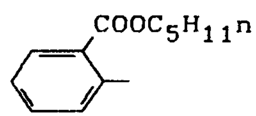 | (2-)$COOCH_3$ | $CH_3$ | $OCH_3$ | 79 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 111 | 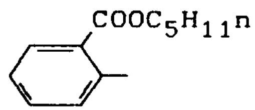 | (2-)$COOC_2H_5$ | $CH_3$ | $OCH_3$ | 115 |
| 112 | 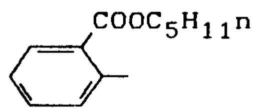 | (2-)$COOC_3H_7n$ | $CH_3$ | $OCH_3$ | 106 |
| 113 | 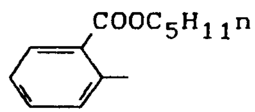 | (2-)$COOCH(CH_3)_2$ | $CH_3$ | $OCH_3$ | 71 |
| 114 | 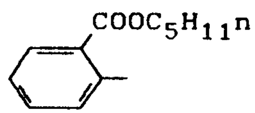 | (2-)$COOCH_2CH_2Cl$ | $CH_3$ | $OCH_3$ | 75 |
| 115 | 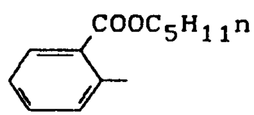 | (2-)$COOCH_2CH_2OCH_3$ | $CH_3$ | $OCH_3$ | 68 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 116 | 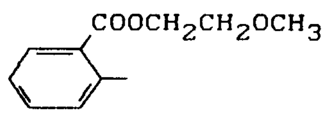 | (2-)$COOCH(CH_3)_2$ | $CH_3$ | $OCH_3$ | 72 |
| 117 | 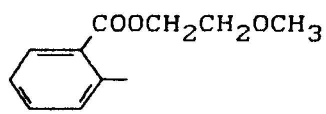 | (2-)$COOCH_2CH_2OCH_3$ | $CH_3$ | $OCH_3$ | 80 |
| 118 | 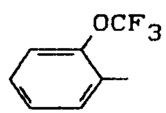 | (2-)$COOCH_3$ | $C_2H_5$ | $OCH_3$ | 78 |
| 119 | 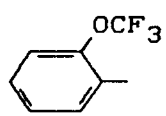 | (2-)$COOC_2H_5$ | $C_2H_5$ | $OCH_3$ | 75 |
| 120 | 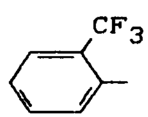 | (2-)$COOCH_3$ | $CH_3$ | $OC_2H_5$ | 98 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 121 | 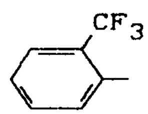 | (2-)$COOC_2H_5$ | $CH_3$ | $OC_2H_5$ | 94 |
| 122 | 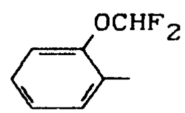 | (2-)$COOCH_3$ | $C_2H_5$ | $OCH_3$ | 93 |
| 123 | 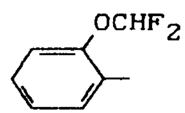 | (2-)$COOC_2H_5$ | $C_2H_5$ | $OCH_3$ | 78 |
| 124 | 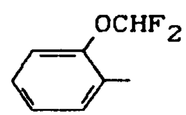 | (2-)$COOCH_3$ | $CH_3$ | $OC_2H_5$ | 92 |
| 125 | 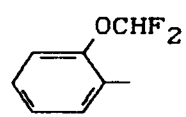 | (2-)$COOC_2H_5$ | $CH_3$ | $OC_2H_5$ | 81 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 126 | 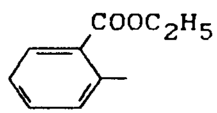 | $(2-)COOCH_3$ | $CH_3$ | $SCH_3$ | 174 |
| 127 | 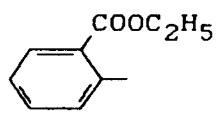 | $(2-)COOC_2H_5$ | $CH_3$ | $SCH_3$ | 82 |
| 128 | 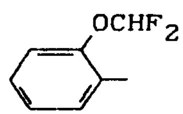 | $(2-)COOCH_3$ | $CH_3$ | $SCH_3$ | 85 |
| 129 | 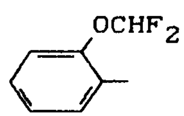 | $(2-)COOC_2H_5$ | $CH_3$ | $SCH_3$ | 80 |
| 130 | 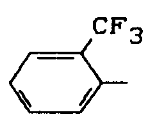 | $(2-)COOCH_3$ | $CH_3$ | $SCH_3$ | 95 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 131 | 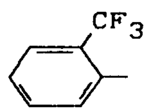 | (2-)$COOC_2H_5$ | $CH_3$ | $SCH_3$ | 120 |
| 132 | 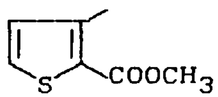 | (2-)$COOCH_3$ | $CH_3$ | $OC_2H_5$ | 98 |
| 133 | 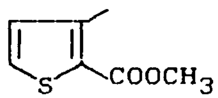 | (2-)$COOC_2H_5$ | $CH_3$ | $OC_2H_5$ | 93 |
| 134 | 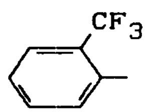 | (2-)$COOCH_3$ | $C_2H_5$ | $OCH_3$ | 84 |
| 135 | 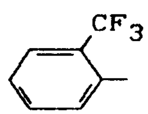 | (2-)$COOC_2H_5$ | $C_2H_5$ | $OCH_3$ | 83 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 136 | 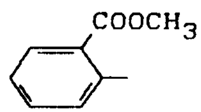 | (2-)$COOCH_3$ | $CH_3$ | $OC_2H_5$ | 157 |
| 137 | 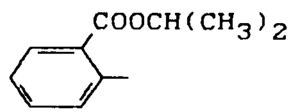 | (2-)$COOC_3H_7n$ | $OCH_3$ | $OCH_3$ | 158 |
| 138 | 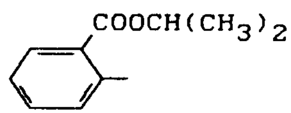 | (2-)$COOCH(CH_3)_2$ | $OCH_3$ | $OCH_3$ | 172 |
| 139 | 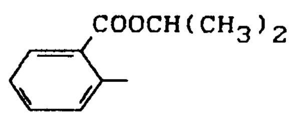 | (2-)$COOCH_2CH_2Cl$ | $OCH_3$ | $OCH_3$ | 156 |
| 140 | 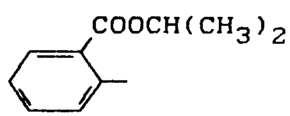 | (2-)$COOCH_2CH_2OCH_3$ | $OCH_3$ | $OCH_3$ | 119 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 141 | 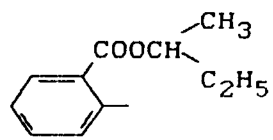 | (2-)$COOCH_3$ | $CH_3$ | $OCH_3$ | 159 |
| 142 | 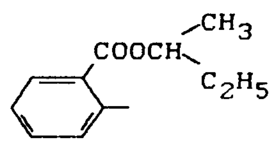 | (2-)$COOC_2H_5$ | $CH_3$ | $OCH_3$ | 141 |
| 143 | 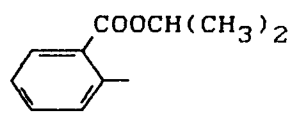 | (2-)$COOC_2H_5$ | $CH_3$ | $OCH_3$ | 158 |
| 144 | 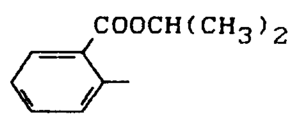 | (2-)$COOC_3H_7n$ | $CH_3$ | $OCH_3$ | 159 |
| 145 | 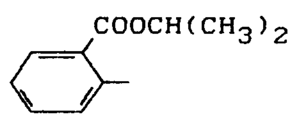 | (2-)$COOCH(CH_3)_2$ | $CH_3$ | $OCH_3$ | 131 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 146 | 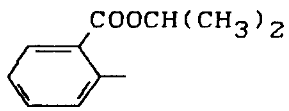 | (2-)$COOCH_2CH_2OCH_3$ | $CH_3$ | $OCH_3$ | 91 |
| 147 | 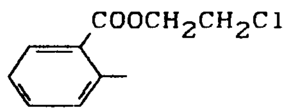 | (2-)$COOC_2H_5$ | $OCH_3$ | $OCH_3$ | 164 |
| 148 | 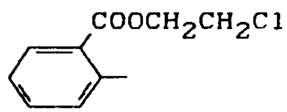 | (2-)$COOC_3H_7n$ | $OCH_3$ | $OCH_3$ | 148 |
| 149 | 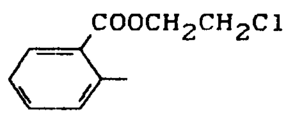 | (2-)$COOCH(CH_3)_2$ | $OCH_3$ | $OCH_3$ | 183 |
| 150 | 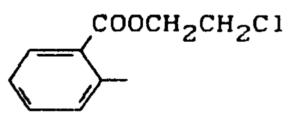 | (2-)$COOCH_2CH_2Cl$ | $OCH_3$ | $OCH_3$ | 156 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 151 | 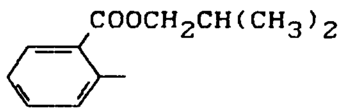 | (2-)$COOCH_3$ | $OCH_3$ | $OCH_3$ | 143 |
| 152 | 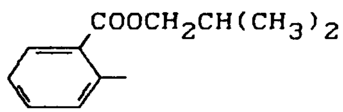 | (2-)$COOC_2H_5$ | $OCH_3$ | $OCH_3$ | 152 |
| 153 | 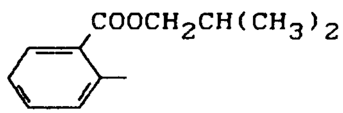 | (2-)$COOCH(CH_3)_2$ | $OCH_3$ | $OCH_3$ | 163 |
| 154 | 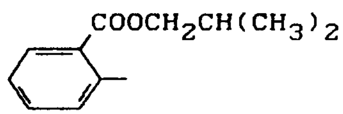 | (2-)$COOCH_2CH_2Cl$ | $OCH_3$ | $OCH_3$ | 168 |
| 155 | 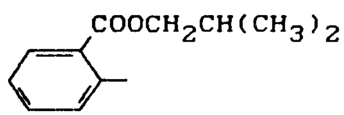 | (2-)$COOCH_2CH_2OCH_3$ | $OCH_3$ | $OCH_3$ | 152 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 156 | 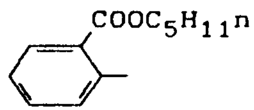 | (2-)$COOCH_3$ | $OCH_3$ | $OCH_3$ | 88 |
| 157 | 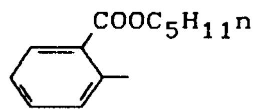 | (2-)$COOC_2H_5$ | $OCH_3$ | $OCH_3$ | 123 |
| 158 | 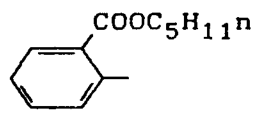 | (2-)$COOC_3H_7n$ | $OCH_3$ | $OCH_3$ | 117 |
| 159 | 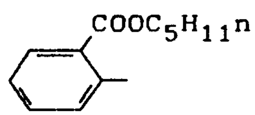 | (2-)$COOCH(CH_3)_2$ | $OCH_3$ | $OCH_3$ | 138 |
| 160 | 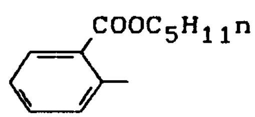 | (2-)$COOCH_2CH_2Cl$ | $OCH_3$ | $OCH_3$ | 104 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 161 | 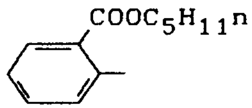 | (2-)$COOCH_2CH_2OCH_3$ | $OCH_3$ | $OCH_3$ | 106 |
| 162 | 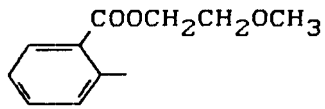 | (2-)$COOC_3H_7n$ | $OCH_3$ | $OCH_3$ | 140 |
| 163 | 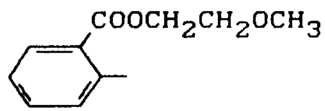 | (2-)$COOCH(CH_3)_2$ | $OCH_3$ | $OCH_3$ | 149 |
| 164 | 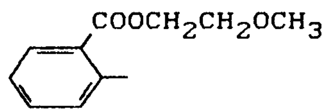 | (2-)$COOCH_2CH_2Cl$ | $OCH_3$ | $OCH_3$ | 117 |
| 165 | 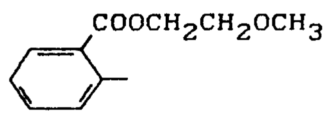 | (2-)$COOCH_2CH_2OCH_3$ | $OCH_3$ | $OCH_3$ | 116 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 166 | 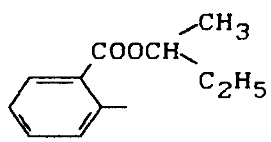 | (2-)$COOC_3H_7n$ | $CH_3$ | $OCH_3$ | 140 |
| 167 | 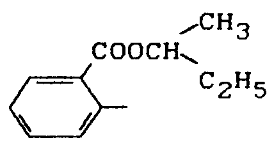 | (2-)$COOCH_3$ | $OCH_3$ | $OCH_3$ | 128 |
| 168 | 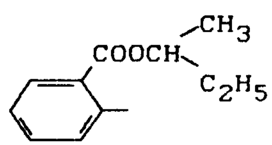 | (2-)$COOC_2H_5$ | $OCH_3$ | $OCH_3$ | 147 |
| 169 | 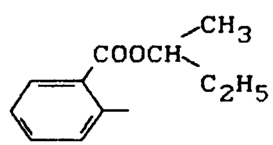 | (2-)$COOC_3H_7n$ | $OCH_3$ | $OCH_3$ | 155 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 170 | 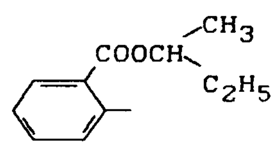 | (2-)$COOCH(CH_3)_2$ | $OCH_3$ | $OCH_3$ | 141 |
| 171 | 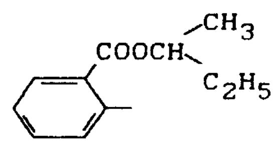 | (2-)$COOCH_2CH_2Cl$ | $OCH_3$ | $OCH_3$ | 118 |
| 172 | 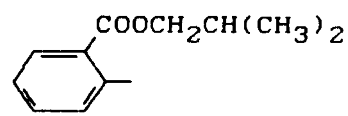 | (2-)$COOCH_3$ | $CH_3$ | $OCH_3$ | 139 |
| 173 | 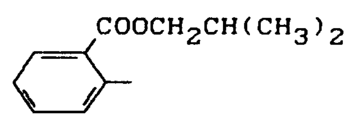 | (2-)$COOC_2H_5$ | $CH_3$ | $OCH_3$ | 158 |
| 174 | 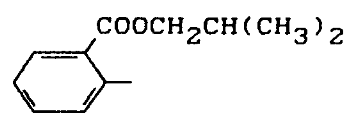 | (2-)$COOC_3H_7n$ | $CH_3$ | $OCH_3$ | 176 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 175 | 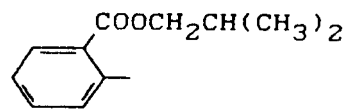 | (2-)$COOCH(CH_3)_2$ | $CH_3$ | $OCH_3$ | 149 |
| 176 | 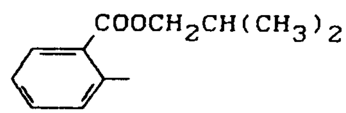 | (2-)$COOCH_2CH_2Cl$ | $CH_3$ | $OCH_3$ | 130 |
| 177 | 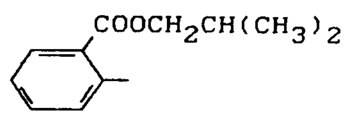 | (2-)$COOCH_2CH_2OCH_3$ | $CH_3$ | $OCH_3$ | 138 |
| 178 | 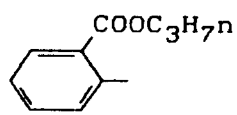 | (2-)$COOC_2H_5$ | $OCH_3$ | $OCH_3$ | 140 |
| 179 | 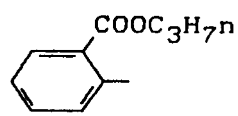 | (2-)$COOC_3H_7n$ | $OCH_3$ | $OCH_3$ | 174 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 180 | 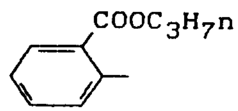 | (2-)$COOCH(CH_3)_2$ | $OCH_3$ | $OCH_3$ | 146 |
| 181 | 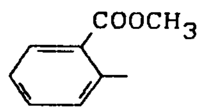 | (2-)$COOCH(CH_3)_2$ | $OCH_3$ | $OCH_3$ | 101 |
| 182 | 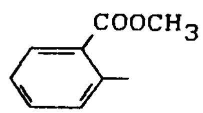 | (2-)$COOCH_2CH_2OCH_3$ | $OCH_3$ | $OCH_3$ | 90 |
| 183 | 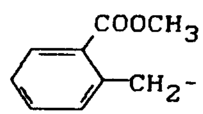 | (2-)$COOCH_2CH_2OCH_3$ | $OCH_3$ | $OCH_3$ | 84 |
| 184 | 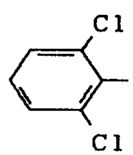 | (2-)$COOCH_3$ | $C_2H_5$ | $OCH_3$ | 93 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 185 | 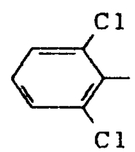 | (2-)$COOC_2H_5$ | $C_2H_5$ | $OCH_3$ | 121 |
| 186 | 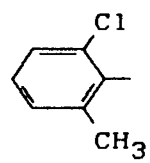 | (2-)$COOCH_3$ | $C_2H_5$ | $OCH_3$ | 86 |
| 187 | 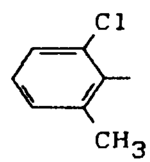 | (2-)$COOC_2H_5$ | $C_2H_5$ | $OCH_3$ | 84 |
| 188 | 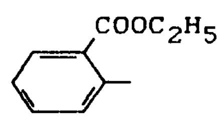 | (2-)$COOC_2H_5$ | $C_2H_5$ | $OCH_3$ | 78 |
| 189 | 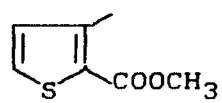 | (2-)$COOCH_3$ | $C_2H_5$ | $OCH_3$ | 76 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 190 | 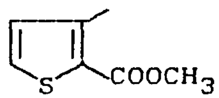 | (2-)$COOC_2H_5$ | $C_2H_5$ | $OCH_3$ | 81 |
| 191 | 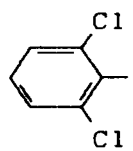 | (2-)$COOC_2H_5$ | $CH_3$ | $OC_2H_5$ | 72 |
| 192 | 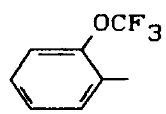 | (2-)$COOCH_3$ | $CH_3$ | $OC_2H_5$ | 76 |
| 193 | 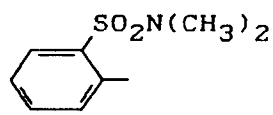 | (2-)$COOC_2H_5$ | $C_2H_5$ | $OCH_3$ | 99 |
| 194 | 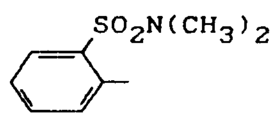 | (2-)$COOCH_3$ | $CH_3$ | $SCH_3$ | 118 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 195 | 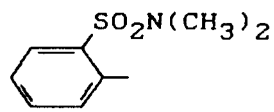 | $(2-)COOC_2H_5$ | $CH_3$ | $SCH_3$ | 112 |
| 196 | 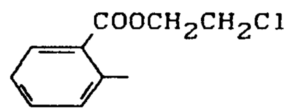 | $(2-)COOCH_2CH_2OCH_3$ | $OCH_3$ | $OCH_3$ | 85 |
| 197 | 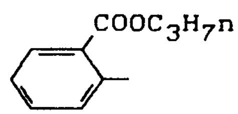 | $(2-)COOCH_2CH_2Cl$ | $OCH_3$ | $OCH_3$ | 76 |
| 198 | 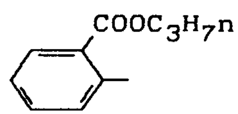 | $(2-)COOCH_2CH_2OCH_3$ | $OCH_3$ | $OCH_3$ | 80 |
| 199 | 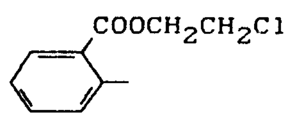 | $(2-)COOCH_2CH_2OCH_3$ | $CH_3$ | $OCH_3$ | 82 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 200 | 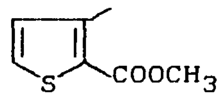 | (2-)$COOCH(CH_3)_2$ | $OCH_3$ | $OCH_3$ | 93 |
| 201 | 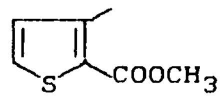 | (2-)$COOCH_2CH_2Cl$ | $OCH_3$ | $OCH_3$ | 87 |
| 202 | 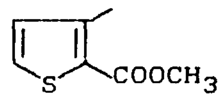 | (2-)$COOCH_2CH_2OCH_3$ | $OCH_3$ | $OCH_3$ | 94 |
| 203 | 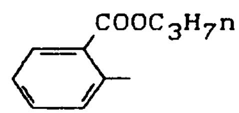 | (2-)$COOCH_2CH_2OCH_3$ | $CH_3$ | $OCH_3$ | 81 |
| 204 | 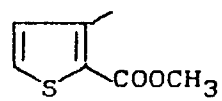 | (2-)$COOCH_3$ | $CH_3$ | $SCH_3$ | 109 |
| 205 | 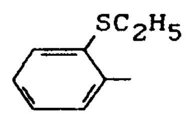 | (2-)$COOCH_3$ | $OCH_3$ | $OCH_3$ | 187 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 206 | 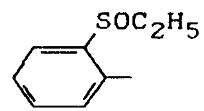 | (2-)$COOCH_3$ | $OCH_3$ | $OCH_3$ | 160 |
| 207 | 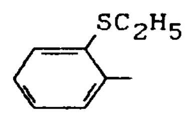 | (2-)$COOCH_3$ | $CH_3$ | $OCH_3$ | 178 |
| 208 | 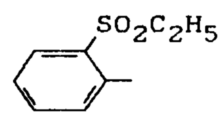 | (2-)$COOCH_3$ | $OCH_3$ | $OCH_3$ | 208 |
| 209 | 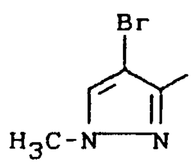 | (2-)$COOCH_3$ | $OCH_3$ | $OCH_3$ | 133 |
| 210 | 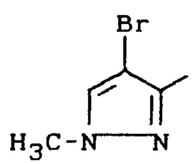 | (2-)$COOC_2H_5$ | $OCH_3$ | $OCH_3$ | 176 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 211 | 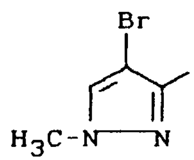 | (2-)$COOC_3H_7n$ | $OCH_3$ | $OCH_3$ | 203 |
| 212 | 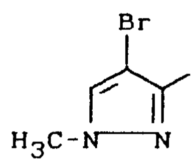 | (2-)$COOCH(CH_3)_2$ | $OCH_3$ | $OCH_3$ | 186 |
| 213 | 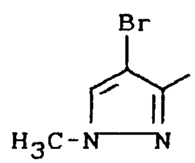 | (2-)$COOC_4H_9n$ | $OCH_3$ | $OCH_3$ | 188 |
| 214 | 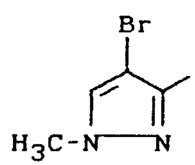 | (2-)$COOCH_2CH(CH_3)_2$ | $OCH_3$ | $OCH_3$ | 172 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 215 | 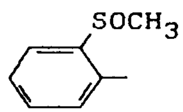 | (2-)$COOCH_3$ | $CH_3$ | $OCH_3$ | |
| 216 | 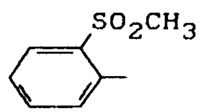 | (2-)$COOCH_3$ | $CH_3$ | $OCH_3$ | |
| 217 | 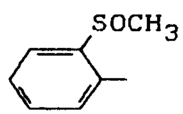 | (2-)$COOC_2H_5$ | $CH_3$ | $OCH_3$ | |
| 218 | 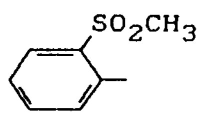 | (2-)$COOC_2H_5$ | $CH_3$ | $OCH_3$ | |
| 219 | 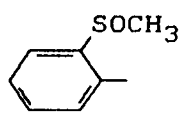 | (2-)$COOCH_3$ | $OCH_3$ | $OCH_3$ | |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 220 | 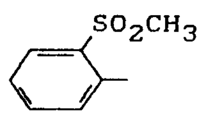 | $(2-)COOCH_3$ | $OCH_3$ | $OCH_3$ | |
| 221 | 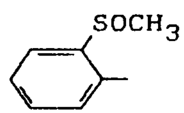 | $(2-)COOC_2H_5$ | $OCH_3$ | $OCH_3$ | |
| 222 | 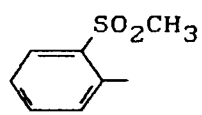 | $(2-)COOC_2H_5$ | $OCH_3$ | $OCH_3$ | |
| 223 | 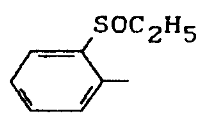 | $(2-)COOCH_3$ | $CH_3$ | $OCH_3$ | |
| 224 | 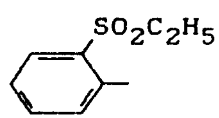 | $(2-)COOCH_3$ | $CH_3$ | $OCH_3$ | 178 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 225 | 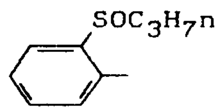 | (2-)$COOCH_3$ | $CH_3$ | $OCH_3$ | |
| 226 | 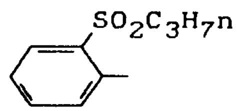 | (2-)$COOCH_3$ | $CH_3$ | $OCH_3$ | |
| 227 | 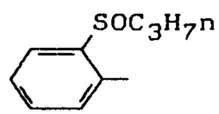 | (2-)$COOCH_3$ | $OCH_3$ | $OCH_3$ | |
| 228 | 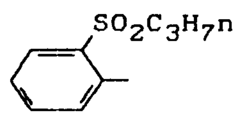 | (2-)$COOCH_3$ | $OCH_3$ | $OCH_3$ | |
| 229 | 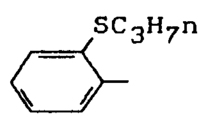 | (2-)$COOCH_3$ | $CH_3$ | $OCH_3$ | |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 230 | 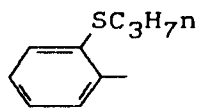 | (2-)$COOCH_3$ | $OCH_3$ | $OCH_3$ | |
| 231 | 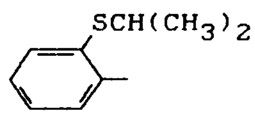 | (2-)$COOCH_3$ | $CH_3$ | $OCH_3$ | |
| 232 | 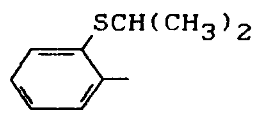 | (2-)$COOCH_3$ | $OCH_3$ | $OCH_3$ | |
| 233 | 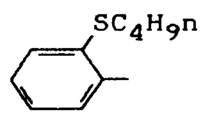 | (2-)$COOCH_3$ | $CH_3$ | $OCH_3$ | |
| 234 | 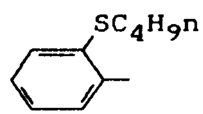 | (2-)$COOCH_3$ | $OCH_3$ | $OCH_3$ | |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 235 | 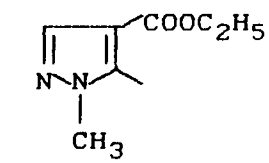 | (2-)$COOCH_3$ | $CH_3$ | $CH_3$ | 143 |
| 236 | 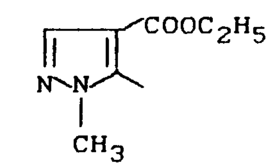 | (2-)$COOC_2H_5$ | $CH_3$ | $CH_3$ | 150 |
| 237 | 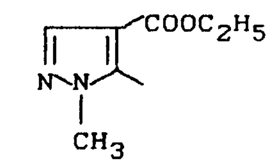 | (2-)$COOC_3H_7n$ | $CH_3$ | $CH_3$ | 140 |
| 238 | 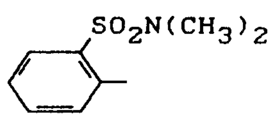 | (2-)$COOC_3H_7n$ | $CH_3$ | $OCH_3$ | 120 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 239 | 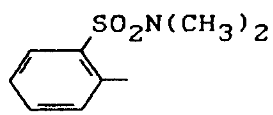 | $(2-)COOCH(CH_3)_2$ | $CH_3$ | $OCH_3$ | 142 |
| 240 | 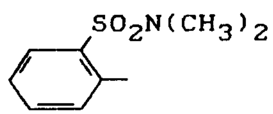 | $(2-)COOC_4H_9n$ | $CH_3$ | $OCH_3$ | 88 |
| 241 | 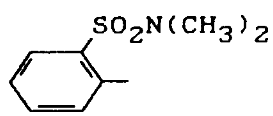 | $(2-)COOCH_2CH(CH_3)_2$ | $CH_3$ | $OCH_3$ | 108 |
| 242 | 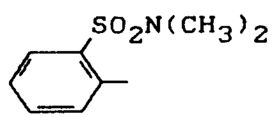 | $(2-)COOCHC_2H_5$<br>$\quad\quad\quad\;|$<br>$\quad\quad\quad CH_3$ | $CH_3$ | $OCH_3$ | 112 |
| 243 | 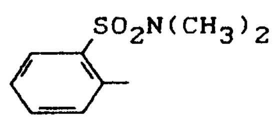 | $(2-)COOC_5H_{11}n$ | $CH_3$ | $OCH_3$ | 93 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 244 | 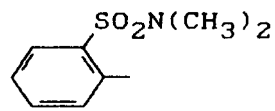 | (2-)$COOCH_2CH_2Cl$ | $CH_3$ | $OCH_3$ | 95 |
| 245 | 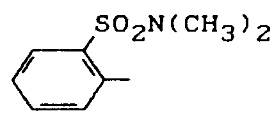 | (2-)$COOCH_2CH_2OCH_3$ | $CH_3$ | $OCH_3$ | 136 |
| 246 | 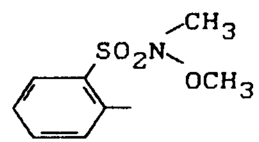 | (2-)$COOC_3H_7n$ | $CH_3$ | $OCH_3$ | 162 |
| 247 | 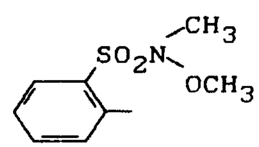 | (2-)$COOCH(CH_3)_2$ | $CH_3$ | $OCH_3$ | 158 |
| 248 | 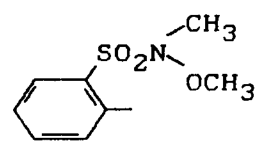 | (2-)$COOC_4H_9n$ | $CH_3$ | $OCH_3$ | 172 |

Tabelle 1 - Fortsetzung

| Bsp.-Nr. | $R^1$ | Position -$COR^2$ | $R^3$ | $R^4$ | Schmelzpunkt (°C) |
|---|---|---|---|---|---|
| 249 | 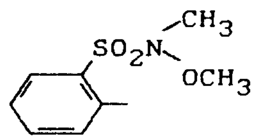 | (2-)$COOCH_2CH(CH_3)_2$ | $CH_3$ | $OCH_3$ | 170 |
| 250 | 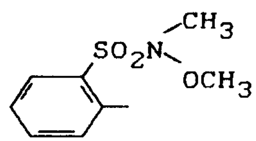 | (2-)$COOCHC_2H_5$<br>$CH_3$ | $CH_3$ | $OCH_3$ | 145 |
| 251 | 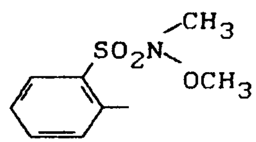 | (2-)$COOC_5H_{11}n$ | $CH_3$ | $OCH_3$ | 140 |
| 252 | 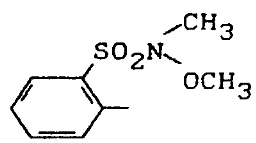 | (2-)$COOCH_2CH_2Cl$ | $CH_3$ | $OCH_3$ | 177 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 253 | 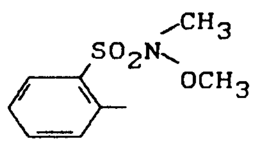 | (2-)$COOCH_2CH_2OCH_3$ | $CH_3$ | $OCH_3$ | 90 |
| 254 | 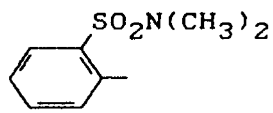 | (2-)$COOCH_2CH_2OCH_3$ | $OCH_3$ | $OCH_3$ | 136 |
| 255 | 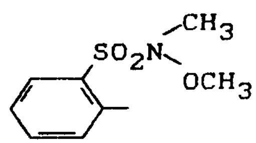 | (2-)$COOCH_2CH_2OCH_3$ | $OCH_3$ | $OCH_3$ | 158 |
| 256 | 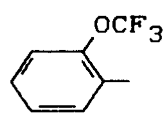 | (2-)$COOCH_3$ | $CH_3$ | $SCH_3$ | 104 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 257 | 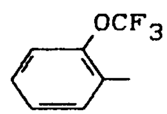 | (2-)$COOC_2H_5$ | $CH_3$ | $SCH_3$ | 78 |
| 258 | 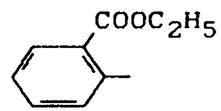 | (2-)COOH | $CH_3$ | $OCH_3$ | 142 |
| 259 | 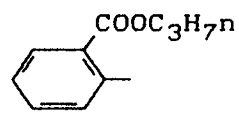 | (2-)COOH | $CH_3$ | $OCH_3$ | (amorph) |
| 260 | 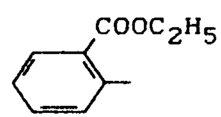 | (2-)$COOCH_3$ | $C_2H_5$ | $OCH_3$ | 141 |
| 261 | 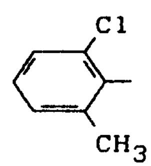 | (2-)$COOCH_3$ | $CH_3$ | $SCH_3$ | 183 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 262 | 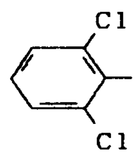 | (2-)$COOCH_3$ | $CH_3$ | $OC_2H_5$ | 103 |
| 263 | 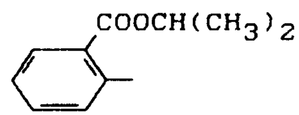 | (2-)$COOCH_2CH_2Cl$ | $CH_3$ | $OCH_3$ | 109 |
| 264 | 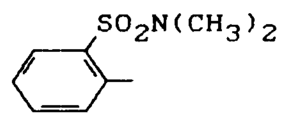 | (2-)$COOCH_3$ | $C_2H_5$ | $OCH_3$ | 104 |
| 265 | 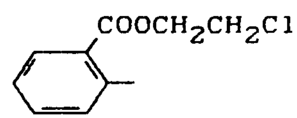 | (2-)$COOCH(CH_3)_2$ | $CH_3$ | $OCH_3$ | 108 |
| 266 | 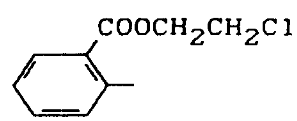 | (2-)$COOCH_2CH_2Cl$ | $CH_3$ | $OCH_3$ | 120 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 267 | 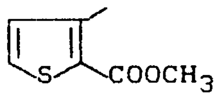 | (2-)$COOC_3H_7n$ | $OCH_3$ | $OCH_3$ | 161 |
| 268 | 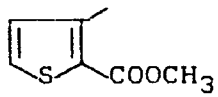 | (2-)$COOC_2H_5$ | $CH_3$ | $SCH_3$ | 110 |
| 269 | 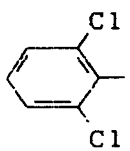 | (2-)$COOCH_3$ | $CH_3$ | $SCH_3$ | 105 |
| 270 | 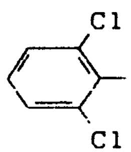 | (2-)$COOC_2H_5$ | $CH_3$ | $SCH_3$ | 100 |
| 271 | 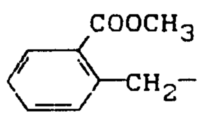 | (2-)$COOCH_2CH_2Cl$ | $CH_3$ | $OCH_3$ | 156 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 272 | 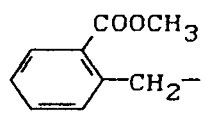 | $(2-)COOCH_2CH_2OCH_3$ | $CH_3$ | $OCH_3$ | 87 |
| 273 | 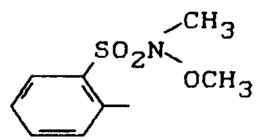 | $(2-)COOCH_3$ | $CH_3$ | $OC_2H_5$ | 113 |
| 274 | 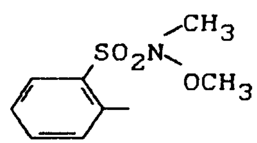 | $(2-)COOC_2H_5$ | $CH_3$ | $OC_2H_5$ | 114 |
| 275 | 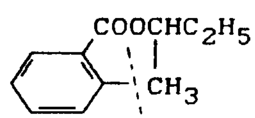 | $(2-)COOCH_2CH_2OCH_3$ | $OCH_3$ | $OCH_3$ | 90 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 276 | 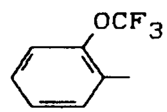 | (2-)$COOC_2H_5$ | $CH_3$ | $OC_2H_5$ | 92 |
| 277 | 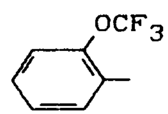 | (2-)$COOC_2H_5$ | $CH_3$ | $OCH_3$ | 127 |
| 278 | 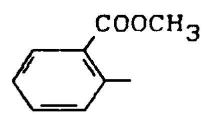 | (2-)$COOC_2H_5$ | $C_2H_5$ | $OCH_3$ | 89 |
| 279 | 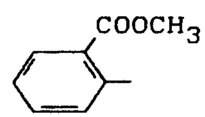 | (2-)$COOCH_3$ | $C_2H_5$ | $OCH_3$ | 106 |
| 280 | 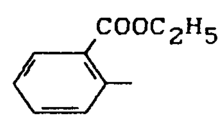 | (2-)$COOC_2H_5$ | $CH_3$ | $OC_2H_5$ | 93 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 281 | 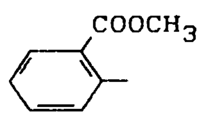 | (2-)$COOC_2H_5$ | $CH_3$ | $OC_2H_5$ | 105 |
| 282 | 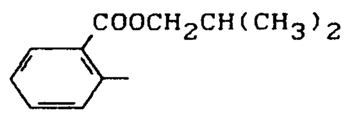 | (2-)$COOC_3H_7n$ | $OCH_3$ | $OCH_3$ | 107 |
| 283 | 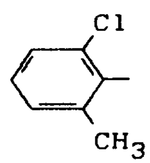 | (2-)$COOCH_3$ | $CH_3$ | $OC_2H_5$ | 108 |
| 284 | 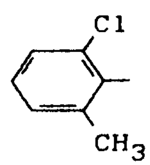 | (2-)$COOC_2H_5$ | $CH_3$ | $OC_2H_5$ | 103 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 285 | 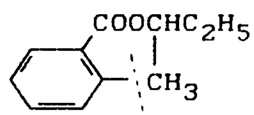 | $(2-)COOCH(CH_3)_2$ | $CH_3$ | $OCH_3$ | 99 |
| 286 | 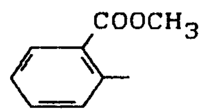 | $(2-)COOCH(CH_3)_2$ | $CH_3$ | $OCH_3$ | 120 |
| 287 | 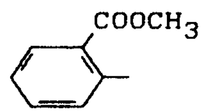 | $(2-)COOCH_2CH_2Cl$ | $CH_3$ | $OCH_3$ | 151 |
| 288 | 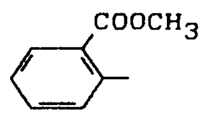 | $(2-)COOCH_2CH_2OCH_3$ | $CH_3$ | $OCH_3$ | 105 |
| 289 | 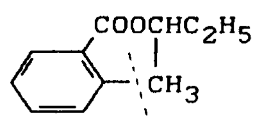 | $(2-)COOCH_2CH_2Cl$ | $CH_3$ | $OCH_3$ | 106 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 290 | 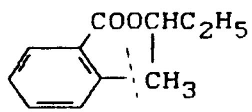 | (2-)$COOCH_2CH_2OCH_3$ | $CH_3$ | $OCH_3$ | 100 |
| 291 | 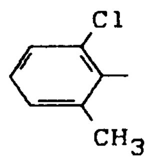 | (2-)$COOC_2H_5$ | $CH_3$ | $SCH_3$ | 112 |
| 292 | 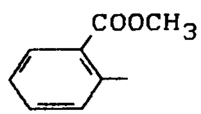 | (2-)$COOC_5H_{11}n$ | $OCH_3$ | $OCH_3$ | 164 |
| 293 | 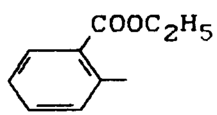 | (2-)$COOC_5H_{11}n$ | $OCH_3$ | $OCH_3$ | 90 |
| 294 | 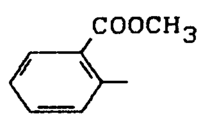 | (2-)$COOC_5H_{11}n$ | $CH_3$ | $OCH_3$ | 162 |

Tabelle 1 - Fortsetzung

| Bsp.-Nr. | $R^1$ | Position $-COR^2$ | $R^3$ | $R^4$ | Schmelzpunkt ($^\circ C$) |
|---|---|---|---|---|---|
| 295 | 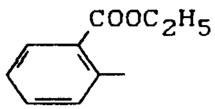 | (2-)$COOC_5H_{11}n$ | $CH_3$ | $OCH_3$ | 140 |
| 296 | 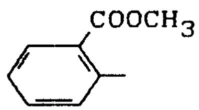 | (2-)$COOCHC_2H_5$<br>\|<br>$CH_3$ | $OCH_3$ | $OCH_3$ | 91 |
| 297 | 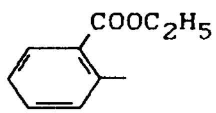 | (2-)$COOCHC_2H_5$<br>\|<br>$CH_3$ | $OCH_3$ | $OCH_3$ | 86 |
| 298 | 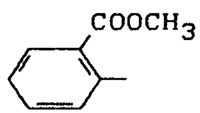 | (2-)$COOCHC_2H_5$<br>\|<br>$CH_3$ | $CH_3$ | $OCH_3$ | 80 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 299 | 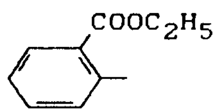 | $(2-)COOCHC_2H_5$<br>$\vert$<br>$CH_3$ | $CH_3$ | $OCH_3$ | 91 |
| 300 | 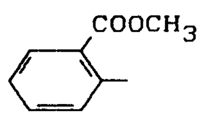 | $(2-)COOCH_2CH(CH_3)_2$ | $OCH_3$ | $OCH_3$ | 108 |
| 301 | 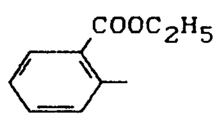 | $(2-)COOCH_2CH(CH_3)_2$ | $OCH_3$ | $OCH_3$ | 181 |
| 302 | 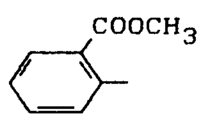 | $(2-)COOCH_2CH(CH_3)_2$ | $CH_3$ | $OCH_3$ | 91 |
| 303 | 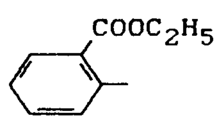 | $(2-)COOCH_2CH(CH_3)_2$ | $CH_3$ | $OCH_3$ | 86 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 304 | 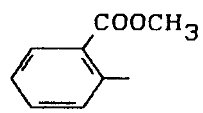 | (2-)$COOC_4H_9n$ | $OCH_3$ | $OCH_3$ | 88 |
| 305 | 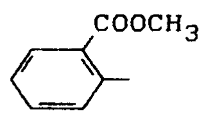 | (2-)$COOC_4H_9n$ | $CH_3$ | $OCH_3$ | 158 |
| 306 | 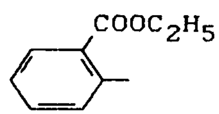 | (2-)$COOC_4H_9n$ | $CH_3$ | $OCH_3$ | 92 |
| 307 | 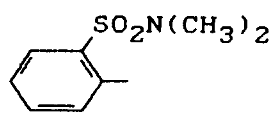 | (2-)$COOCH_3$ | $CH_3$ | $OC_2H_5$ | 190 |
| 308 | 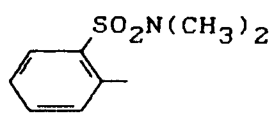 | (2-)$COOC_2H_5$ | $CH_3$ | $OC_2H_5$ | 104 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 309 | 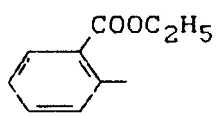 | (2-)$COOCH_3$ | $CH_3$ | $OC_2H_5$ | 85 |
| 310 | 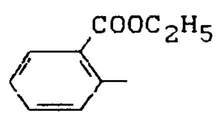 | (2-)$COOC_4H_9n$ | $OCH_3$ | $OCH_3$ | 148 |
| 311 | 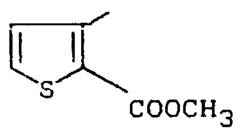 | (2-)$COOC_5H_{11}n$ | $CH_3$ | $OCH_3$ | 91 |
| 312 | 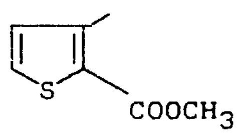 | (2-)$COOC_4H_9n$ | $CH_3$ | $OCH_3$ | 168 |
| 313 | 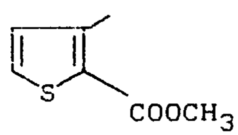 | (2-)$COOCH_2CH(CH_3)_2$ | $CH_3$ | $OCH_3$ | 118 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 314 | 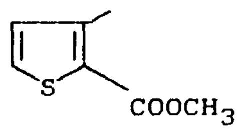 | $(2-)COOC_4H_9n$ | $OCH_3$ | $OCH_3$ | 97 |
| 315 | 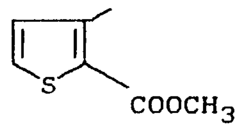 | $(2-)COOCH_2CH(CH_3)_2$ | $OCH_3$ | $OCH_3$ | 105 |
| 316 | 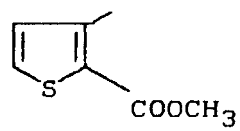 | $(2-)COOCHC_2H_5$<br>$CH_3$ | $OCH_3$ | $OCH_3$ | 103 |
| 317 | 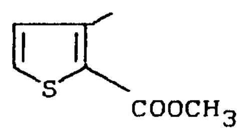 | $(2-)COOC_5H_{11}n$ | $OCH_3$ | $OCH_3$ | 90 |
| 318 | 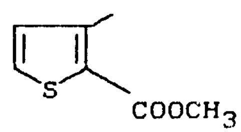 | $(2-)COOCHC_2H_5$<br>$CH_3$ | $CH_3$ | $OCH_3$ | 108 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 319 | 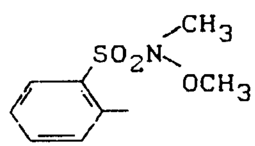 | (2-)$COOC_3H_7n$ | $OCH_3$ | $OCH_3$ | 172 |
| 320 | 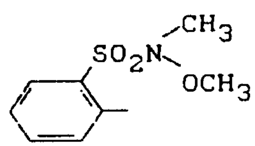 | (2-)$COOCH(CH_3)_2$ | $OCH_3$ | $OCH_3$ | 183 |
| 321 | 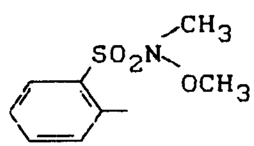 | (2-)$COOC_4H_9n$ | $OCH_3$ | $OCH_3$ | 166 |
| 322 | 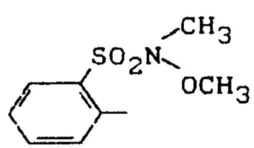 | (2-)$COOCH_2CH(CH_3)_2$ | $OCH_3$ | $OCH_3$ | 174 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 323 | 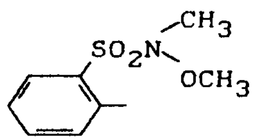 | (2-)$COOCH_2CH_2Cl$ | $OCH_3$ | $OCH_3$ | 180 |
| 324 | 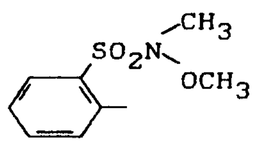 | (2-)$COOCHC_2H_5$<br>$CH_3$ | $OCH_3$ | $OCH_3$ | 93 |
| 325 | 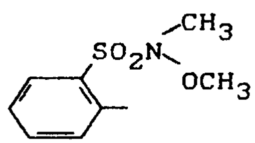 | (2-)$COOC_5H_{11}n$ | $OCH_3$ | $OCH_3$ | 136 |
| 326 | 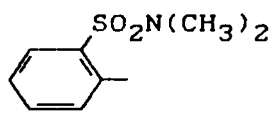 | (2-)$COOC_3H_7n$ | $OCH_3$ | $OCH_3$ | 106 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 327 | 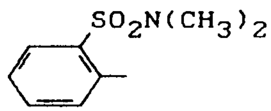 | (2-)$COOCH(CH_3)_2$ | $OCH_3$ | $OCH_3$ | 112 |
| 328 | 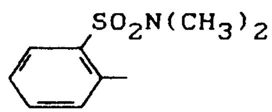 | (2-)$COOC_4H_9n$ | $OCH_3$ | $OCH_3$ | 104 |
| 329 | 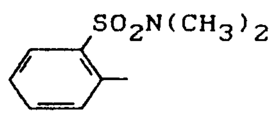 | (2-)$COOCH_2CH(CH_3)_2$ | $OCH_3$ | $OCH_3$ | 134 |
| 330 | 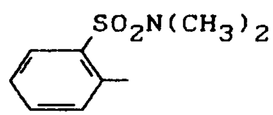 | (2-)$COOCHC_2H_5$<br>$CH_3$ | $OCH_3$ | $OCH_3$ | 163 |
| 331 | 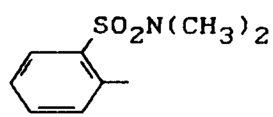 | (2-)$COOC_5H_{11}n$ | $OCH_3$ | $OCH_3$ | 106 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 332 | 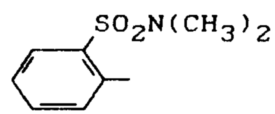 | (2-)$COOCH_2CH_2Cl$ | $OCH_3$ | $OCH_3$ | 119 |
| 333 | 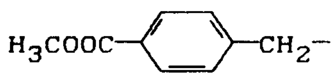 | (2-)$COOCH_3$ | $OCH_3$ | $OCH_3$ | 190 |
| 334 | 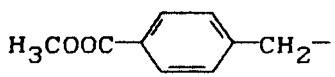 | (2-)$COOC_2H_5$ | $OCH_3$ | $OCH_3$ | 195 |
| 335 | 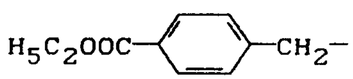 | (2-)$COOCH_3$ | $OCH_3$ | $OCH_3$ | 178 |
| 336 | 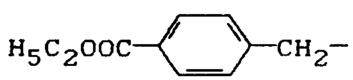 | (2-)$COOC_2H_5$ | $OCH_3$ | $OCH_3$ | 189 |
| 337 | 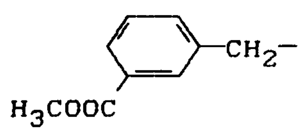 | (2-)$COOC_2H_5$ | $CH_3$ | $OCH_3$ | 91 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 338 | 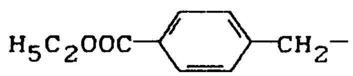 | (2-)$COOCH_3$ | $CH_3$ | $OCH_3$ | 90 |
| 339 | 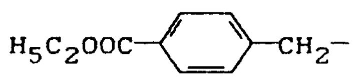 | (2-)$COOC_2H_5$ | $CH_3$ | $OCH_3$ | 87 |
| 340 | 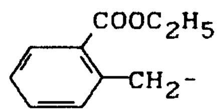 | (2-)$COOC_2H_5$ | $CH_3$ | $OCH_3$ | 80 |
| 341 | 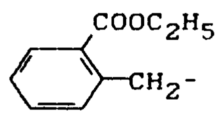 | (2-)$COOCH_3$ | $CH_3$ | $OCH_3$ | 80 |
| 342 | 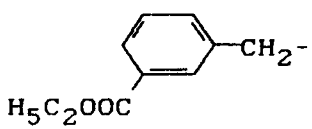 | (2-)$COOC_2H_5$ | $CH_3$ | $OCH_3$ | 83 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 343 | 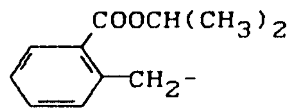 | (2-)$COOCH_3$ | $OCH_3$ | $OCH_3$ | 90 |
| 344 | 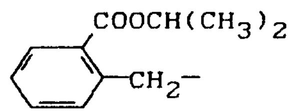 | (2-)$COOC_2H_5$ | $OCH_3$ | $OCH_3$ | 82 |
| 345 | 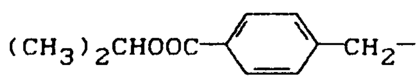 | (2-)$COOCH_3$ | $OCH_3$ | $OCH_3$ | 85 |
| 346 | 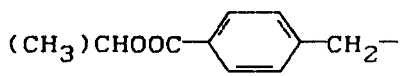 | (2-)$COOC_2H_5$ | $OCH_3$ | $OCH_3$ | 86 |
| 347 | 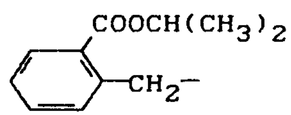 | (2-)$COOCH_3$ | $CH_3$ | $OCH_3$ | 90 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 348 | 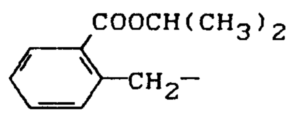 | (2-)$COOC_2H_5$ | $CH_3$ | $OCH_3$ | 92 |
| 349 | 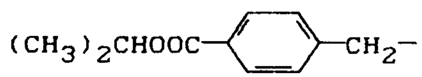 | (2-)$COOCH_3$ | $CH_3$ | $OCH_3$ | 178 |
| 350 | 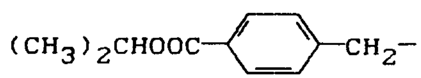 | (2-)$COOC_2H_5$ | $CH_3$ | $OCH_3$ | 162 |
| 351 | 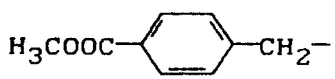 | (2-)$COOCH_3$ | $CH_3$ | $OCH_3$ | 163 |
| 352 | 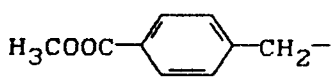 | (2-)$COOC_2H_5$ | $CH_3$ | $OCH_3$ | 165 |
| 353 | 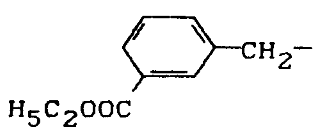 | (2-)$COOCH_3$ | $CH_3$ | $OCH_3$ | 94 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 354 | 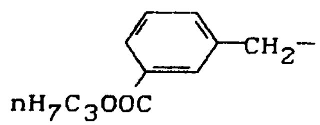 | (2-)$COOC_2H_5$ | $CH_3$ | $OCH_3$ | 92 |
| 355 | 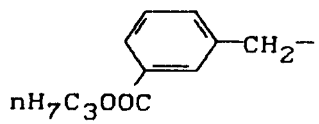 | (2-)$COOCH_3$ | $CH_3$ | $OCH_3$ | 106 |
| 356 | 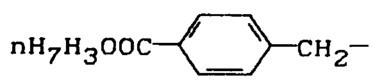 | (2-)$COOC_2H_5$ | $CH_3$ | $OCH_3$ | 87 |
| 357 | 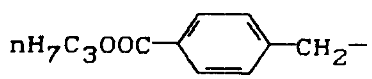 | (2-)$COOCH_3$ | $CH_3$ | $OCH_3$ | 94 |
| 358 | 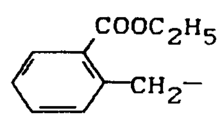 | (2-)$COOCH_3$ | $OCH_3$ | $OCH_3$ | 80 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 359 | 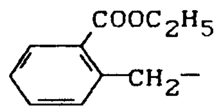 | (2-)$COOC_2H_5$ | $OCH_3$ | $OCH_3$ | 72 |
| 360 | 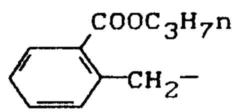 | (2-)$COOCH_3$ | $OCH_3$ | $OCH_3$ | 80 |
| 361 | 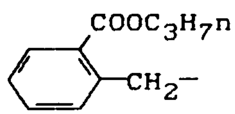 | (2-)$COOC_2H_5$ | $OCH_3$ | $OCH_3$ | 75 |
| 362 | 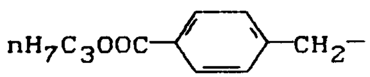 | (2-)$COOCH_3$ | $OCH_3$ | $OCH_3$ | 141 |
| 363 | 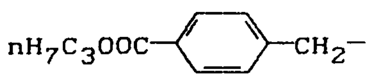 | (2-)$COOC_2H_5$ | $OCH_3$ | $OCH_3$ | 138 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 364 | 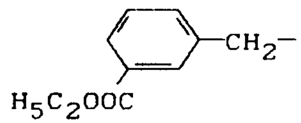 | (2-)$COOCH_3$ | $OCH_3$ | $OCH_3$ | 90 |
| 365 | 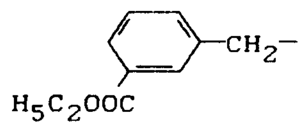 | (2-)$COOC_2H_5$ | $OCH_3$ | $OCH_3$ | 76 |
| 366 | 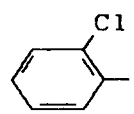 | (2-)$COOCH_3$ | $CH_3$ | $OCH_3$ | 88 |
| 367 | 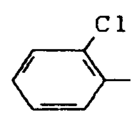 | (2-)$COOC_2H_5$ | $CH_3$ | $OCH_3$ | 81 |
| 368 | 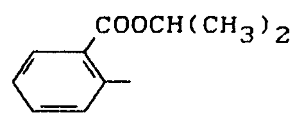 | (2-)COOH | $CH_3$ | $OCH_3$ | 138 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 369 | 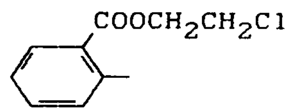 | (2-)COOH | $CH_3$ | $OCH_3$ | 116 |
| 370 | 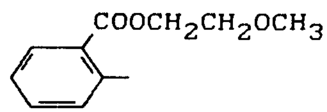 | (2-)COOH | $CH_3$ | $OCH_3$ | 95 |
| 371 | 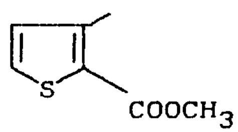 | (2-)COOH | $CH_3$ | $OCH_3$ | 131 |
| 372 | 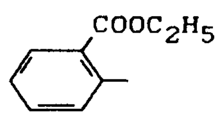 | (2-)COOH | $OCH_3$ | $OCH_3$ | 76 |

Tabelle 1 - Fortsetzung

| Bsp.- Nr. | $R^1$ | Position $-COR^2$ | $R^3$ | $R^4$ | Schmelz- punkt (°C) |
|---|---|---|---|---|---|
| 373 | 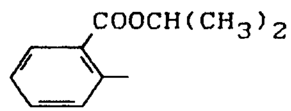 | (2-)COOH | $OCH_3$ | $OCH_3$ | 104 |
| 374 | 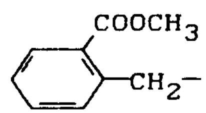 | (2-)COOH | $CH_3$ | $OCH_3$ | 119 |
| 375 | 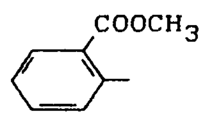 | (2-)COOH | $OCH_3$ | $OCH_3$ | 110 |
| 376 | 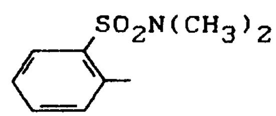 | (2-)COOH | $CH_3$ | $OCH_3$ | 140 |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 377 | 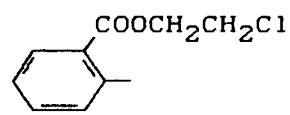 | (2-)COOH | $OCH_3$ | $OCH_3$ | 103 |
| 378 | 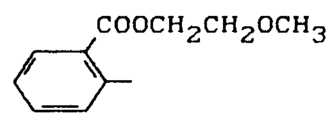 | (2-)COOH | $OCH_3$ | $OCH_3$ | 100 |
| 379 | 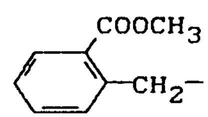 | (2-)COOH | $OCH_3$ | $OCH_3$ | 120 |
| 380 | 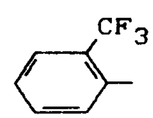 | (2-)COOH | $OCH_3$ | $OCH_3$ | 124 |

<u>Tabelle 1</u> - Fortsetzung

| Bsp.-Nr. | $R^1$ | Position $-COR^2$ | $R^3$ | $R^4$ | Schmelzpunkt (°C) |
|---|---|---|---|---|---|
| 381 | 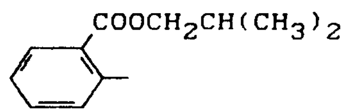 | (2-)COOH | $CH_3$ | $OCH_3$ | 74 |
| 382 | 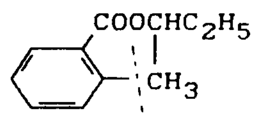 | (2-)COOH | $OCH_3$ | $OCH_3$ | 97 |
| 383 | 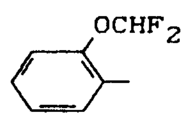 | (2-)COOH | $OCH_3$ | $OCH_3$ | 105 |
| 384 | 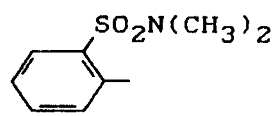 | (2-)COOH | $OCH_3$ | $OCH_3$ | |

Tabelle 1 - Fortsetzung

| Bsp.-Nr. | $R^1$ | Position $-COR^2$ | $R^3$ | $R^4$ | Schmelzpunkt (°C) |
|---|---|---|---|---|---|
| 385 | 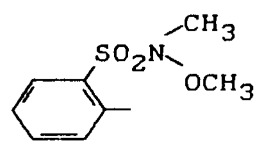 | (2-)COOH | $OCH_3$ | $OCH_3$ | |
| 386 | 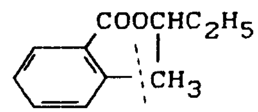 | (2-)COOH | $CH_3$ | $OCH_3$ | |

Ausgangsstoffe der Formel (IV):

Beispiel (IV-1)

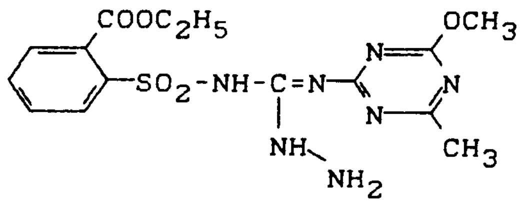

Eine Mischung aus 6,5 g (15,3 mMol) N'-(4-Methoxy-6-methyl-s-triazin-2-yl)-N"-(2-ethoxycarbonylphenylsulfonyl)-S-methyl-isothioharnstoff, 0,77 g (15,3 mMol) Hydrazinhydrat und 150 ml Methylenchlorid wird 30 Minuten bei 20 ° C gerührt, dann mit Magnesiumsulfat und Kieselgel verrührt und abgesaugt. Das Filtrat wird eingeengt, der Rückstand mit Diethylether verrührt und das kristallin angefallene Produkt durch Absaugen isoliert. Man erhält 4,9 g (78 % der Theorie) N'-(4-Methoxy-6-methyl-s-triazin-2-yl)-N"-amino-N"'-(2-ethoxycarbonyl-phenylsulfonyl)-guanidin vom Schmelzpunkt 141 ° C.

Anwendungsbeispiele:

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle, Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 1, 4, 5, 6 und 7 bei guter Verträglichkeit gegenüber Weizen starke Wirkung gegen Unkräuter.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 1, 4, 5, 6 und 7 bei guter Verträglichkeit gegenüber Weizen starke Wirkung gegen Unkräuter.

**Patentansprüche**

**1.** Substituierte Arylsulfonylaminoguanidinotriazine der allgemeinen Formel (I)

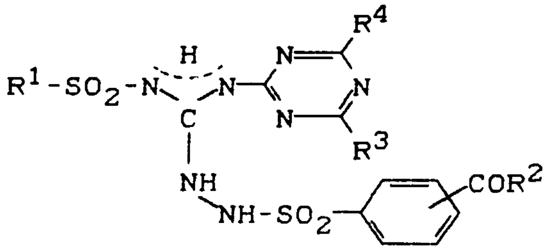

(I)

in welcher

$R^1$ für jeweils gegebenenfalls substituiertes Aryl, Aralkyl oder Heteroaryl steht,

$R^2$ für Hydroxy, Amino oder für jeweils gegebenenfalls substituiertes Alkoxy oder Alkylamino steht,

$R^3$ für Halogen, Alkyl, Halogenalkyl, Alkoxyalkyl, Alkoxy, Halogenalkoxy, Alkylthio, Halogenalkylthio, Alkylamino oder Dialkylamino steht und

$R^4$ für Halogen, Alkyl, Cycloalkyl, Alkoxy, Alkylthio, Alkylamino oder Dialkylamino steht,

ausgenommen die Verbindungen N'-(4,6-Dimethoxy-s-triazin-2-yl)-N''-(2-methoxycarbonyl-phenylsulfonylamino)-N'''-(2-brom-phenylsulfonyl)-guanidin, N'-(4,6-Dimethoxy-s-triazin-2-yl)-N''-(2-methoxycarbonyl-phenylsulfonylamino)-N'''-(2-phenyl-phenylsulfonyl)-guanidin, N'-(4-Methoxy-6-methyl-s-triazin-2-yl)-N''-(2-methoxycarbonyl-phenylsulfonylamino)-N'''-(2-phenyl-phenylsulfonyl)-guanidin, N'-(4,6-Dimethoxy-s-triazin-2-yl)-N''-(2-methoxycarbonyl-phenyl-sulfonylamino)-N'''-(2-methylthiophenylsulfonyl)-guanidin und N-(4,6-Dimethoxy-s-triazin-2-yl)-N''-(2-methoxycarbonyl-phenylsulfonylamino)-N'''-(2-isopropoxycarbonyl-phenylsulfonyl)-guanidin.

**2.** Verfahren zur Herstellung von substituierten Arylsulfonylaminoguanidinotriazinen der Formel (I) gemäß Anspruch 1, dadurch gekennzeichnet, daß man

(a) Sulfonylverbindungen der allgemeinen Formel (II)

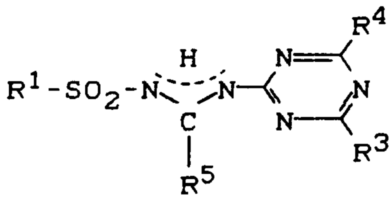

(II)

in welcher

$R^1$, $R^3$ und $R^4$ die in Anspruch 1 angegebenen Bedeutungen haben und

$R^5$ für eine der nachstehend angegebenen Abgangsgruppen

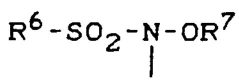

oder $-Q-R^8$ steht,

worin

$R^6$ die oben für $R^1$ angegebene Bedeutung hat, aber nicht in jedem Einzelfall mit $R^1$ identisch sein muß,

$R^7$ für Alkyl, Alkenyl oder Aralkyl steht,

$R^8$ für Alkyl, Aralkyl oder Aryl steht und

Q für Sauerstoff oder Schwefel steht,

mit Sulfonsäurehydraziden der allgemeinen Formel (III)

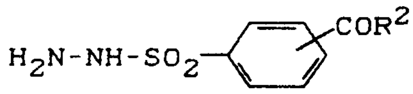 (III)

in welcher

$R^2$ die in Anspruch 1 angegebene Bedeutung hat,

gegebenenfalls in Gegenwart eines Verdünnungsmittels umsetzt, oder daß man

(b) Aminoguanidinotriazine der allgemeinen Formel (IV)

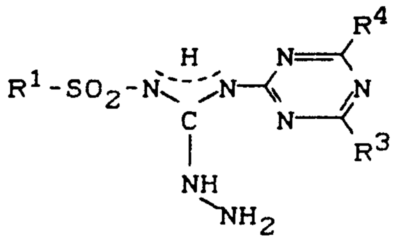 (IV)

in welcher

$R^1$, $R^3$ und $R^4$ die in Anspruch 1 angegebenen Bedeutungen haben,

mit Sulfonsäurehalogeniden der allgemeinen Formel (V)

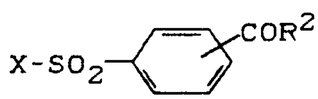 (V)

in welcher

$R^2$ die in Anspruch 1 angegebene Bedeutung hat und

X für Halogen steht,

oder mit Sulfobenzoesäureanhydrid der Formel (VI)

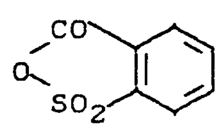 (VI)

gegebenenfalls in Gegenwart eines Verdünnungsmittels und gegebenenfalls in Gegenwart eines Säureakzeptors umsetzt, oder daß man

(c) substituierte Arylsulfonylaminoguanidinotriazine der allgemeinen Formel (I), in welcher $R^2$ für gegebenenfalls substituiertes Alkoxy steht und $R^1$, $R^3$ und $R^4$ die oben angegebenen Bedeutungen haben,

mit wäßrigen Alkalilaugen oder mit Ammoniak oder mit gegebenenfalls substituierten Alkylaminen gegebenenfalls in Gegenwart eines Verdünnungsmittels umsetzt.

**3.** Herbizide Mittel, gekennzeichnet durch einen Gehalt an mindestens einem substituierten Arylsulfonylaminoguanidinotriazin der Formel (I) gemäß Anspruch 1.

**4.** Verfahren zur Bekämpfung von Unkräutern, dadurch gekennzeichnet, daß man substituierte Arylsulfonylaminoguanidinotriazine der Formel (I) gemäß Anspruch 1 auf Unkräuter und/oder ihren Lebensraum einwirken läßt.

**5.** Verwendung von substituierten Arylsulfonylaminoguanidinotriazinen der Formel (I) gemäß Anspruch 1

zur Bekämpfung von Unkräutern.

**6.** Verfahren zur Herstellung von herbiziden Mitteln, dadurch gekennzeichnet, daß man substituierte Arylsulfonylaminoguanidinotriazine der Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 92 10 1511

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | EP-A-0 302 378 (BAYER AG) 8. Februar 1989<br>* das ganze Dokument *<br>--- | 1-6 | C07D251/16<br>A01N47/44<br>C07D403/12 |
| D,P,X | EP-A-0 414 067 (BAYER AG) 27. Februar 1991<br>* das ganze Dokument *<br>----- | 1-6 | C07D251/46<br>C07D251/42<br>C07D409/12 |

RECHERCHIERTE SACHGEBIETE (Int. Cl.5 )

C07D

Der vorliegende Recherchenbericht wurde für alle Patentansprüche erstellt

| Recherchenort | Abschlußdatum der Recherche | Prüfer |
|---|---|---|
| DEN HAAG | 12 MAI 1992 | DE JONG B.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)